# EUROPEAN PATENT APPLICATION

(11) **EP 4 029 935 A1**
(43) Date of publication of application: **20.07.2022**
(21) Application number: 21151673.7
(22) Date of filing: 14.01.2021
(51) Int. Cl.: C12N 9/22, C12N 15/04, C12C 11/00, C12G 1/00, C12R 1/86

(54) **S. EUBAYANUS STRAIN AND HYBRIDS THEREOF**

(71) Applicant: Carlsberg A/S, 1799 Copenhagen V (DK)
(72) Inventor: Stovicek, Vratislav, 13000 Prague 3 (CZ); Förster, Jochen, 1799 Copenhagen V (DK)
(74) Representative: Høiberg P/S

(57) **Abstract**

The present disclosure relates to a method of generating novel hybrid yeast strains. In particular, it relates to the mating of a *S*. *eubayanus* yeast cell with a spore of another yeast strain from the genus *Saccharomyces.* The disclosure also relates to a *S*. *eubayanus* yeast cell comprising a mutant HO endonuclease, and a hybrid yeast strain, where one of the parental strains is said *S*. *eubayanus* yeast. The disclosure also relates to a method of producing a beverage using the hybrid yeast strain of the invention.

SEQUENCE LISTING

<110>  Carlsberg A/S

<120>  S. eubayanus strain and hybrids thereof

<130>  P5841EP00

<160>  10

<170>  PatentIn version 3.5

<210>  1
<211>  1761
<212>  DNA
<213>  Saccharomyces eubayanus

<220>
<221>  misc_feature
<222>  (1)..(1761)
<223>  S. eubayanus HO gene coding region wild type

<400>  1
atgctttccg aaaatacaac tatattgatg gccaatggcg aaattaaaga catcgctaat      60

gtcaccgcta attcctacgt tatgtgcgaa gatggttcag ccgcccgcgt tataagcgtc     120

acacaaggtt gtcaaaaaat ctacaatatt cagcaaaaaa ccaagcatag agcttttgaa     180

ggcgaacctg gtagattgga ccctagacgt agaactattt accaacgcct gaacttgcaa     240

tgtaccgcag gtcataaact atctgtcagg gttcctacta aaccactgtt agagaaaagt     300

ggtagaagtg ccacgaaata caaagtgaga tggagaaact tacaacaatg tcagacactt     360

gacggaagaa taataacaat tccaaaaaat caccacaaaa cgttcccaat gactgtcgaa     420

ggtgaatttg ccgcgaaacg tttcatcgaa gaaatggagc ttttgaaagg cgaatacttt     480

aactttgaca tcgaagtccg agatttggac taccttgatg ctcagttgag aatttcaagc     540

tgtattagat tcagtccggt tattgcaggg aatggtgttt tatctaaatt tctcactgga     600

cgtaatgacc tagtgactcc cgctgtaaaa agtatggctt ggatgcttgg tttatggcta     660

ggtgacggta caacaaaaga acccgagatc tctgtagata gtttggaccc caaactaatg     720

gaaagtctac gagaacaagc caaaatttgg ggcctctacc ttacagtgtg tgatgaccat     780

gttccgctgc gtgccaaaca cgtgaggcta cattatgggg atgggccaga tgaaaatagg     840

aagaccagga atttaaggaa gaataatcca ttttggaacg ccgtcacaaa attgaagttt     900

aaaagggagc tcgatggtga gaagcaaatc ccagagttca tgtacagcga gcatgtagag     960

gtccgtgaag catttttggc aggtttaatc gattcagatg gatacgtcgt gaaaaagggc    1020

gaaggccctg aatcttacaa aatagcaatt caaactgttt attcatcaat tatggacggg    1080

gttgttcata tttcaagatc tcttggaatg tccgctactg tgacgactag atccgctaga    1140

gaagaaatta ttgagggaag gaaagttcaa tgccaattca catatgactg taatgttgct    1200

ggaggaacaa cattacaaaa cgttttatca tattgtcgaa gtggccataa aacgagagaa 1260

attcccccaa ttgtgaaaag aaaaccagtt tatttcgggt tcacggacga ttttcaaggt 1320

gagagtacag tatacggact tcacatagag gggcataaaa gttacttatt aggcaataaa 1380

atagaagtca agtcttgtgg aggctattgt gaaggagaac aacctaaatt atcccagaag 1440

aaaaacttga aacactgtat tgcgtgtcct agaaagggca ttaaatactt ttataaagat 1500

tggagcggta aaaaccgagt gtgtgctaga tgttacggaa gatacaaatt tagtggccat 1560

cactgtataa actgcaaata tgtaccagaa gcacgtgagg tgaaaaaggc gaaagataaa 1620

ggtgaaaaat tgggcataac accagaaggc ctgccgttta aaggaccaga atgtttacga 1680

tgtgggggga ttttgcaatt tgacgctgtt cgtggacccc ataagagttg tgggacaaat 1740

atcggggtac gggtttgttg a 1761

<210> 2
<211> 586
<212> PRT
<213> Saccharomyces eubayanus

<220>
<221> MISC_FEATURE
<222> (1)..(586)
<223> S. eubayanus HO endonuclease protein wild type

<400> 2

Met Leu Ser Glu Asn Thr Thr Ile Leu Met Ala Asn Gly Glu Ile Lys
1               5                   10                  15

Asp Ile Ala Asn Val Thr Ala Asn Ser Tyr Val Met Cys Glu Asp Gly
            20                  25                  30

Ser Ala Ala Arg Val Ile Ser Val Thr Gln Gly Cys Gln Lys Ile Tyr
        35                  40                  45

Asn Ile Gln Gln Lys Thr Lys His Arg Ala Phe Glu Gly Glu Pro Gly
    50                  55                  60

Arg Leu Asp Pro Arg Arg Arg Thr Ile Tyr Gln Arg Leu Asn Leu Gln
65                  70                  75                  80

Cys Thr Ala Gly His Lys Leu Ser Val Arg Val Pro Thr Lys Pro Leu
                85                  90                  95

Leu Glu Lys Ser Gly Arg Ser Ala Thr Lys Tyr Lys Val Arg Trp Arg
            100                 105                 110

Asn Leu Gln Gln Cys Gln Thr Leu Asp Gly Arg Ile Ile Thr Ile Pro
        115                 120                 125

Lys Asn His His Lys Thr Phe Pro Met Thr Val Glu Gly Glu Phe Ala
    130                 135                 140

Ala Lys Arg Phe Ile Glu Glu Met Glu Leu Leu Lys Gly Glu Tyr Phe
145                 150                 155                 160

Asn Phe Asp Ile Glu Val Arg Asp Leu Asp Tyr Leu Asp Ala Gln Leu
                165                 170                 175

Arg Ile Ser Ser Cys Ile Arg Phe Ser Pro Val Ile Ala Gly Asn Gly
            180                 185                 190

Val Leu Ser Lys Phe Leu Thr Gly Arg Asn Asp Leu Val Thr Pro Ala
        195                 200                 205

Val Lys Ser Met Ala Trp Met Leu Gly Leu Trp Leu Gly Asp Gly Thr
    210                 215                 220

Thr Lys Glu Pro Glu Ile Ser Val Asp Ser Leu Asp Pro Lys Leu Met
225                 230                 235                 240

Glu Ser Leu Arg Glu Gln Ala Lys Ile Trp Gly Leu Tyr Leu Thr Val
                245                 250                 255

Cys Asp Asp His Val Pro Leu Arg Ala Lys His Val Arg Leu His Tyr
            260                 265                 270

Gly Asp Gly Pro Asp Glu Asn Arg Lys Thr Arg Asn Leu Arg Lys Asn
        275                 280                 285

Asn Pro Phe Trp Asn Ala Val Thr Lys Leu Lys Phe Lys Arg Glu Leu
    290                 295                 300

Asp Gly Glu Lys Gln Ile Pro Glu Phe Met Tyr Ser Glu His Val Glu
305                 310                 315                 320

Val Arg Glu Ala Phe Leu Ala Gly Leu Ile Asp Ser Asp Gly Tyr Val
                325                 330                 335

Val Lys Lys Gly Glu Gly Pro Glu Ser Tyr Lys Ile Ala Ile Gln Thr
            340                 345                 350

Val Tyr Ser Ser Ile Met Asp Gly Val Val His Ile Ser Arg Ser Leu
        355                 360                 365

Gly Met Ser Ala Thr Val Thr Thr Arg Ser Ala Arg Glu Glu Ile Ile
    370                 375                 380

Glu Gly Arg Lys Val Gln Cys Gln Phe Thr Tyr Asp Cys Asn Val Ala
385                 390                 395                 400

Gly Gly Thr Thr Leu Gln Asn Val Leu Ser Tyr Cys Arg Ser Gly His
                405                 410                 415

Lys Thr Arg Glu Ile Pro Pro Ile Val Lys Arg Lys Pro Val Tyr Phe
            420                 425                 430

Gly Phe Thr Asp Asp Phe Gln Gly Glu Ser Thr Val Tyr Gly Leu His
        435                 440                 445

Ile Glu Gly His Lys Ser Tyr Leu Leu Gly Asn Lys Ile Glu Val Lys
    450                 455                 460

Ser Cys Gly Gly Tyr Cys Glu Gly Glu Gln Pro Lys Leu Ser Gln Lys
465                 470                 475                 480

Lys Asn Leu Lys His Cys Ile Ala Cys Pro Arg Lys Gly Ile Lys Tyr
                485                 490                 495

Phe Tyr Lys Asp Trp Ser Gly Lys Asn Arg Val Cys Ala Arg Cys Tyr
            500                 505                 510

Gly Arg Tyr Lys Phe Ser Gly His His Cys Ile Asn Cys Lys Tyr Val
        515                 520                 525

Pro Glu Ala Arg Glu Val Lys Lys Ala Lys Asp Lys Gly Glu Lys Leu
    530                 535                 540

Gly Ile Thr Pro Glu Gly Leu Pro Phe Lys Gly Pro Glu Cys Leu Arg
545                 550                 555                 560

Cys Gly Gly Ile Leu Gln Phe Asp Ala Val Arg Gly Pro His Lys Ser
                565                 570                 575

Cys Gly Thr Asn Ile Gly Val Arg Val Cys
            580                 585

<210>  3
<211>  1761
<212>  DNA
<213>  Saccharomyces eubayanus

<220>
<221>  misc_feature
<222>  (1)..(1761)
<223>  S. eubayanus HO gene C238T mutation

<400>  3
atgctttccg aaaatacaac tatattgatg gccaatggcg aaattaaaga catcgctaat      60

gtcaccgcta attcctacgt tatgtgcgaa gatggttcag ccgcccgcgt tataagcgtc     120

acacaaggtt gtcaaaaaat ctacaatatt cagcaaaaaa ccaagcatag agcttttgaa     180

ggcgaacctg gtagattgga ccctagacgt agaactattt accaacgcct gaacttgtaa     240

tgtaccgcag gtcataaact atctgtcagg gttcctacta aaccactgtt agagaaaagt     300

ggtagaagtg ccacgaaata caaagtgaga tggagaaact tacaacaatg tcagacactt     360

gacggaagaa taataacaat tccaaaaaat caccacaaaa cgttcccaat gactgtcgaa     420

ggtgaatttg ccgcgaaacg tttcatcgaa gaaatggagc ttttgaaagg cgaatacttt     480

aactttgaca tcgaagtccg agatttggac taccttgatg ctcagttgag aatttcaagc     540

tgtattagat tcagtccggt taytgcaggg aatggtgttt tatctaaatt tctcactgga     600

cgtaatgacc tagtgactcc cgctgtaaaa agtatggctt ggatgcttgg tttatggcta     660

ggtgacggta caacaaaaga acccgagatc tctgtagata gtttggaccc caaactaatg     720

gaaagtctac gagaacaagc caaaatttgg ggcctctacc ttacagtgtg tgatgaccat     780

gttccgctgc gtgccaaaca cgtgaggcta cattatgggg atgggccaga tgaaaatagg     840

aagaccagga atttaaggaa gaataatcca ttttggaacg ccgtcacaaa attgaagttt     900

aaaagggagc tcgatggtga gaagcaaatc ccagagttca tgtacagcga gcatgtagag     960

gtccgtgaag catttttggc aggtttaatc gattcagatg gatacgtcgt gaaaaagggc    1020

gaaggccctg aatcttacaa aatagcaatt caaactgttt attcatcaat tatggacggg    1080

gttgttcata tttcaagatc tcttggaatg tccgctactg tgacgactag atccgctaga    1140

gaagaaatta ttgagggaag gaaagttcaa tgccaattca catatgactg taatgttgct    1200

ggaggaacaa cattacaaaa cgttttatca tattgtcgaa gtggccataa aacgagagaa    1260

attcccccaa ttgtgaaaag aaaaccagtt tatttcgggt tcacggacga ttttcaaggt    1320

gagagtacag tatacggact tcacatagag gggcataaaa gttacttatt aggcaataaa    1380

atagaagtca agtcttgtgg aggctattgt gaaggagaac aacctaaatt atcccagaag    1440

aaaaacttga aacactgtat tgcgtgtcct agaaagggca ttaaatactt ttataaagat    1500

tggagcggta aaaaccgagt gtgtgctaga tgttacggaa gatacaaatt tagtggccat    1560

cactgtataa actgcaaata tgtaccagaa gcacgtgagg tgaaaaaggc gaaagataaa    1620

ggtgaaaaat tgggcataac accagaaggc ctgccgttta aaggaccaga atgtttacga    1680

tgtgggggga ttttgcaatt tgacgctgtt cgtggacccc ataagagttg tgggacaaat    1740

atcggggtac gggtttgttg a                                              1761

<210>  4
<211>  1512
<212>  DNA
<213>  Saccharomyces eubayanus

<220>
<221>  misc_feature
<222>  (1)..(1512)
<223>  S. eubayanus FDC1-like gene coding region wild type

<400>  4
atgagtcaat tacaaccagc tttgaaattt agagacttta ttcaagtgct caaggatgag      60

gatgatctag ttgaaatcac tgaagaggtc gattcaaatc tagaggtagg tgctatcatg     120

agaaaagcgt atgaatctca cttaccagct ccactattta ggaacgttaa aggcgcttcg     180

aaggatcttt ttagcatttt aggttgccca gccggcttga gaagtaaaga gaaaggtgat     240

catggcagaa ttgctcatca tctcgggctc gacccaaaga caccgatcaa agaaatcata     300

gactacttgc tagaatgtaa agacaaggaa ccattgcctc caaagatagt ttctgtgtca     360

tctgctcctt gcaaggaaca cgtcctctct gaaggtgaag ttcatttaga tagtcttcca     420

gcaccatact tgcatacttc tgatggtggc aagtatttgc aaacgtatgg aatgtggatt     480

cttcaaactc cagataagaa gtggactaat tggtccattg ctagaggtat ggttgtcgac     540

gacaagcata tcacaggtct ggtaattaaa ccacaacata ttagacaaat tgctgatgct     600

tgggcagcca tcggtaaagg agatgaaatt ccatttgcgc tatgttttgg tgttcctcca     660

gcagctatct tagtcagttc aatgccaatt cctgaaggtg aatctgaatc tgattatatc     720

ggtgcaattt tgggcgaggc agtgccggta gttaagtgtg aaaccaacga cttgttggtt     780

cctgcaacaa gcgaaatggt ttttgagggt acattgtcct taaagcaaac ccatatcgaa     840

ggcccattcg gcgaaatgca tggatacgtt tttgagggac aggctcatcc gtgtccaatg     900

tatactgtca aagccatgac atacagagac aacgccattt tacctgtatc aaacccaggt     960

atctgtacgg acgaaactca tactttgatt ggttcattag tggctaccga agccaagaag    1020

cttgctatag attctggctt accagttctc gacgctttta ctccatacga agctcaagct    1080

ttgtggttgg ttttgaaggt ggatttgaag aagctgcaag cactaaagac tacacccgag    1140

gagttctgca agaaggttgg tgaaatattt tttagatcga aagttggatt catcatccat    1200

gagatagttc tagtcgcaga agatatcgac atattcaatt tcaaggaggt tttctgggct    1260

tatgttacca gacatacccc tgttgcagac cagatgactt tcgatgacgt cacctctttc    1320

cctttggctc cctttgtttc ccagtcacct agaagtaaga ctatgaaagg tgggaagtgt    1380

gttacgaact gcatttttag acagcaatat gagcgcgatt ttgaatacgt cacttgtaat    1440

tttgaaaagg ggtattcgaa ggaattagtt gataaagtta acgaaaattg gaagacgtat     1500

ggctacaagt ga                                                         1512

<210>  5
<211>  503
<212>  PRT
<213>  Saccharomyces eubayanus

<220>
<221>  MISC_FEATURE
<222>  (1)..(503)
<223>  S. eubayanus FDC1-like protein wild type

<400>  5

Met Ser Gln Leu Gln Pro Ala Leu Lys Phe Arg Asp Phe Ile Gln Val
1               5                   10                  15

Leu Lys Asp Glu Asp Asp Leu Val Glu Ile Thr Glu Glu Val Asp Ser
            20                  25                  30

Asn Leu Glu Val Gly Ala Ile Met Arg Lys Ala Tyr Glu Ser His Leu
        35                  40                  45

Pro Ala Pro Leu Phe Arg Asn Val Lys Gly Ala Ser Lys Asp Leu Phe
    50                  55                  60

Ser Ile Leu Gly Cys Pro Ala Gly Leu Arg Ser Lys Glu Lys Gly Asp
65                  70                  75                  80

His Gly Arg Ile Ala His His Leu Gly Leu Asp Pro Lys Thr Pro Ile
                85                  90                  95

Lys Glu Ile Ile Asp Tyr Leu Leu Glu Cys Lys Asp Lys Glu Pro Leu
            100                 105                 110

Pro Pro Lys Ile Val Ser Val Ser Ser Ala Pro Cys Lys Glu His Val
        115                 120                 125

Leu Ser Glu Gly Glu Val His Leu Asp Ser Leu Pro Ala Pro Tyr Leu
    130                 135                 140

His Thr Ser Asp Gly Gly Lys Tyr Leu Gln Thr Tyr Gly Met Trp Ile
145                 150                 155                 160

Leu Gln Thr Pro Asp Lys Lys Trp Thr Asn Trp Ser Ile Ala Arg Gly
                165                 170                 175

Met Val Val Asp Asp Lys His Ile Thr Gly Leu Val Ile Lys Pro Gln
            180                 185                 190

His Ile Arg Gln Ile Ala Asp Ala Trp Ala Ala Ile Gly Lys Gly Asp
        195                 200                 205

Glu Ile Pro Phe Ala Leu Cys Phe Gly Val Pro Pro Ala Ala Ile Leu
    210                 215                 220

Val Ser Ser Met Pro Ile Pro Glu Gly Glu Ser Glu Ser Asp Tyr Ile
225                 230                 235                 240

Gly Ala Ile Leu Gly Glu Ala Val Pro Val Val Lys Cys Glu Thr Asn
                245                 250                 255

Asp Leu Leu Val Pro Ala Thr Ser Glu Met Val Phe Glu Gly Thr Leu
            260                 265                 270

Ser Leu Lys Gln Thr His Ile Glu Gly Pro Phe Gly Glu Met His Gly
        275                 280                 285

Tyr Val Phe Glu Gly Gln Ala His Pro Cys Pro Met Tyr Thr Val Lys
    290                 295                 300

Ala Met Thr Tyr Arg Asp Asn Ala Ile Leu Pro Val Ser Asn Pro Gly
305                 310                 315                 320

Ile Cys Thr Asp Glu Thr His Thr Leu Ile Gly Ser Leu Val Ala Thr
                325                 330                 335

Glu Ala Lys Lys Leu Ala Ile Asp Ser Gly Leu Pro Val Leu Asp Ala
            340                 345                 350

Phe Thr Pro Tyr Glu Ala Gln Ala Leu Trp Leu Val Leu Lys Val Asp
        355                 360                 365

Leu Lys Lys Leu Gln Ala Leu Lys Thr Thr Pro Glu Glu Phe Cys Lys
    370                 375                 380

Lys Val Gly Glu Ile Phe Phe Arg Ser Lys Val Gly Phe Ile Ile His
385                 390                 395                 400

Glu Ile Val Leu Val Ala Glu Asp Ile Asp Ile Phe Asn Phe Lys Glu
                405                 410                 415

Val Phe Trp Ala Tyr Val Thr Arg His Thr Pro Val Ala Asp Gln Met
            420                 425                 430

Thr Phe Asp Asp Val Thr Ser Phe Pro Leu Ala Pro Phe Val Ser Gln
        435                 440                 445

Ser Pro Arg Ser Lys Thr Met Lys Gly Gly Lys Cys Val Thr Asn Cys
    450                 455                 460

Ile Phe Arg Gln Gln Tyr Glu Arg Asp Phe Glu Tyr Val Thr Cys Asn
465                 470                 475                 480

Phe Glu Lys Gly Tyr Ser Lys Glu Leu Val Asp Lys Val Asn Glu Asn
                485                 490                 495

Trp Lys Thr Tyr Gly Tyr Lys
            500

<210>  6
<211>  1512
<212>  DNA
<213>  Saccharomyces cerevisiae

<220>
<221>  misc_feature
<222>  (1)..(1512)
<223>  S. cerevisiae FDC1 like gene wild type

<400>  6
atgaggaagc taaatccagc tttagaattt agagacttta tccaggtctt aaaagatgaa      60

gatgacttaa tcgaaattac cgaagagatt gatccaaatc tcgaagtagg tgcaattatg     120

aggaaggcct atgaatccca cttaccagcc ccgttattta aaaatctcaa aggtgcttcg     180

aaggatcttt tcagcatttt aggttgccca gccggtttga gaagtaagga gaaaggagat     240

catggtagaa ttgcccatca tctggggctc gacccaaaaa caactatcaa ggaaatcata     300

gattatttgc tggagtgtaa ggagaaggaa cctctccccc caatcactgt tcctgtgtca     360

tctgcacctt gtaaaacaca tatactttct gaagaaaaaa tacatctaca aagcctgcca     420

acaccatatc tacatgtttc agacggtggc aagtacttac aaacgtacgg aatgtggatt     480

cttcaaactc cagataaaaa atggactaat tggtcaattg ctagaggtat ggttgtagat     540

gacaagcata tcactggtct ggtaattaaa ccacaacata ttagacaaat tgctgactct     600

tgggcagcaa ttggaaaagc aaatgaaatt cctttcgcgt tatgttttgg cgttccccca     660

gcagctattt tagttagttc catgccaatt cctgaaggtg tttctgaatc ggattatgtt     720

ggcgcaatct tgggtgagtc ggttccagta gtaaaatgtg agaccaacga tttaatggtt     780

cctgcaacga gtgagatggt atttgagggt actttgtcct taacagatac acatctggaa     840

ggcccatttg gtgagatgca tggatatgtt ttcaaaagcc aaggtcatcc ttgtccattg     900

tacactgtca aggctatgag ttacagagac aatgctattc tacctgtttc gaaccccggt 960

ctttgtacgg atgagacaca taccttgatt ggttcactag tggctactga ggccaaggag 1020

ctggctattg aatctggctt gccaattctg gatgccttta tgccttatga ggctcaggct 1080

ctttggctta tcttaaaggt ggatttgaaa gggctgcaag cattgaagac aacgcctgaa 1140

gaattttgta agaaggtagg tgatatttac tttaggacaa aagttggttt tatagtccat 1200

gaaataattt tggtggcaga tgatatcgac atatttaact tcaaagaagt catctgggcc 1260

tacgttacaa gacatacacc tgttgcagat cagatggctt ttgatgatgt cacttctttt 1320

cctttggctc cctttgtttc gcagtcatcc agaagtaaga ctatgaaagg tggaaagtgc 1380

gttactaatt gcatatttag acagcaatat gagcgcagtt ttgactacat aacttgtaat 1440

tttgaaaagg gatatccaaa aggattagtt gacaaagtaa atgaaaattg gaaaaggtac 1500

ggatataaat aa 1512

<210> 7
<211> 503
<212> PRT
<213> Saccharomyces cerevisiae

<220>
<221> MISC_FEATURE
<222> (1)..(503)
<223> S. cerevisiae FDC1-like protein wild type

<400> 7

Met Arg Lys Leu Asn Pro Ala Leu Glu Phe Arg Asp Phe Ile Gln Val
1               5                   10                  15

Leu Lys Asp Glu Asp Asp Leu Ile Glu Ile Thr Glu Glu Ile Asp Pro
            20                  25                  30

Asn Leu Glu Val Gly Ala Ile Met Arg Lys Ala Tyr Glu Ser His Leu
        35                  40                  45

Pro Ala Pro Leu Phe Lys Asn Leu Lys Gly Ala Ser Lys Asp Leu Phe
    50                  55                  60

Ser Ile Leu Gly Cys Pro Ala Gly Leu Arg Ser Lys Glu Lys Gly Asp
65                  70                  75                  80

His Gly Arg Ile Ala His His Leu Gly Leu Asp Pro Lys Thr Thr Ile
                85                  90                  95

Lys Glu Ile Ile Asp Tyr Leu Leu Glu Cys Lys Glu Lys Glu Pro Leu
            100                 105                 110

Pro Pro Ile Thr Val Pro Val Ser Ser Ala Pro Cys Lys Thr His Ile
        115                 120                 125

Leu Ser Glu Glu Lys Ile His Leu Gln Ser Leu Pro Thr Pro Tyr Leu
    130                 135                 140

His Val Ser Asp Gly Gly Lys Tyr Leu Gln Thr Tyr Gly Met Trp Ile
145                 150                 155                 160

Leu Gln Thr Pro Asp Lys Lys Trp Thr Asn Trp Ser Ile Ala Arg Gly
                165                 170                 175

Met Val Val Asp Asp Lys His Ile Thr Gly Leu Val Ile Lys Pro Gln
            180                 185                 190

His Ile Arg Gln Ile Ala Asp Ser Trp Ala Ala Ile Gly Lys Ala Asn
        195                 200                 205

Glu Ile Pro Phe Ala Leu Cys Phe Gly Val Pro Pro Ala Ala Ile Leu
    210                 215                 220

Val Ser Ser Met Pro Ile Pro Glu Gly Val Ser Glu Ser Asp Tyr Val
225                 230                 235                 240

Gly Ala Ile Leu Gly Glu Ser Val Pro Val Val Lys Cys Glu Thr Asn
                245                 250                 255

Asp Leu Met Val Pro Ala Thr Ser Glu Met Val Phe Glu Gly Thr Leu
            260                 265                 270

Ser Leu Thr Asp Thr His Leu Glu Gly Pro Phe Gly Glu Met His Gly
        275                 280                 285

Tyr Val Phe Lys Ser Gln Gly His Pro Cys Pro Leu Tyr Thr Val Lys
    290                 295                 300

Ala Met Ser Tyr Arg Asp Asn Ala Ile Leu Pro Val Ser Asn Pro Gly
305                 310                 315                 320

Leu Cys Thr Asp Glu Thr His Thr Leu Ile Gly Ser Leu Val Ala Thr
                325                 330                 335

Glu Ala Lys Glu Leu Ala Ile Glu Ser Gly Leu Pro Ile Leu Asp Ala
            340                 345                 350

Phe Met Pro Tyr Glu Ala Gln Ala Leu Trp Leu Ile Leu Lys Val Asp
        355                 360                 365

Leu Lys Gly Leu Gln Ala Leu Lys Thr Thr Pro Glu Glu Phe Cys Lys
    370                 375                 380

Lys Val Gly Asp Ile Tyr Phe Arg Thr Lys Val Gly Phe Ile Val His
385                 390                 395                 400

Glu Ile Ile Leu Val Ala Asp Asp Ile Asp Ile Phe Asn Phe Lys Glu
                405                 410                 415

Val Ile Trp Ala Tyr Val Thr Arg His Thr Pro Val Ala Asp Gln Met
            420                 425                 430

Ala Phe Asp Asp Val Thr Ser Phe Pro Leu Ala Pro Phe Val Ser Gln
        435                 440                 445

Ser Ser Arg Ser Lys Thr Met Lys Gly Gly Lys Cys Val Thr Asn Cys
    450                 455                 460

Ile Phe Arg Gln Gln Tyr Glu Arg Ser Phe Asp Tyr Ile Thr Cys Asn
465                 470                 475                 480

Phe Glu Lys Gly Tyr Pro Lys Gly Leu Val Asp Lys Val Asn Glu Asn
                485                 490                 495

Trp Lys Arg Tyr Gly Tyr Lys
            500

<210>  8
<211>  230
<212>  DNA
<213>  Saccharomyces eubayanus

<220>
<221>  misc_feature
<222>  (1)..(230)
<223>  Target sequence for S. eubayanus HO gene

<400>  8
aaggttgtca aaaaatctac aatattcagc aaaaaaccaa gcatagagct tttgaaggcg      60

aacctggtag attggaccct agacgtagaa ctatttacca acgcctgaac ttgtaatgta     120

ccgcaggtca taaactatct gtcagggttc ctactaaacc actgttagag aaaagtggta     180

gaagtgccac gaaatacaaa gtgagatgga gaaacttaca acaatgtcag                230

<210>  9
<211>  226
<212>  DNA
<213>  Saccharomyces eubayanus

<220>
<221>  misc_feature
<222>  (1)..(226)
<223>  Target sequence for S. eubayanus FDC1-like gene

<400>  9
tgcaaggaac acgtcctctc tgaaggtgaa gttcatttag atagtcttcc agcaccatac      60

ttgcatactt ctgatggtgg caagtatttg caaacgtatg gaatgtgaat tcttcaaact     120

ccagataaga agtggactaa ttggtccatt gctagaggta tggttgtcga cgacaagcat     180

atcacaggtc tggtaattaa accacaacat attagacaaa ttgctg                    226


<210>  10
<211>  374
<212>  DNA
<213>  Saccharomyces cerevisiae


<220>
<221>  misc_feature
<222>  (1)..(374)
<223>  Target sequence for S. cerevisae FDC1 gene

<400>  10
ccaatcactg ttcctgtgtc atctgcacct tgtaaaacac atatactttc tgaagaaaaa      60

atacatctac aaagcctgcc aacaccatat ctacatgttt cagacggtgg caagtactta     120

caaacgtacg gaatgtggat tcttcaaact ccagataaaa aatggactaa ttgatcaatt     180

gctagaggta tggttgtaga tgacaagcat atcactggtc tggtaattaa accacaacat     240

attagacaaa ttgctgactc ttgggcagca attggaaaag caaatgaaat tcctttcgcg     300

ttatgttttg gcgttccccc agcagctatt ttagttagtt ccatgccaat tcctgaaggt     360

gtttctgaat cgga                                                       374

## Description

### Technical field

The present invention relates to a method of generating novel hybrid yeast strains. In particular, it relates to the mating of a heterothallic *S. eubayanus* yeast cell with a spore of another yeast strain from the genus *Saccharomyces.* The invention also relates to a *S. eubayanus* yeast cell comprising a mutant HO endonuclease, and a hybrid yeast strain, where one of the parental strains is said *S. eubayanus* yeast.

### Background

The yeast strains currently used for production of lager beer, *Saccharomyces pastorianus* (*S. pastorianus*), are hybrids of *S. cerevisiae* and *S. eubayanus.* The long domestication of the current lager yeast strains has resulted in limited genotypic and thus phenotypic diversity. In particular, the flavor and aroma diversity produced by *S. pastorianus* is low. In addition, some interesting features may have been lost over time.

Breeding of different strains and thus combining different traits may constitute a way to bring more diversity into the *S. pastorianus* production strain portfolio. However, the alloploid nature and multiple hybridization events of the species resulted in genomic changes (chromosomal rearrangements and aneuploidy) leading to low sporulation capabilities and/or limited spore viability (Gorter de Vries *et al.,* 2019). This has made it difficult to combine new traits within currently existing domesticated strains and to select for new phenotypes in a predictable manner, even though such attempts have been documented (Sanchez *et al.,* 2012). Furthermore, interspecies hybridization occurs at a relatively low rate; reported hybridization frequencies range from 1.5 to 3.6% for mass mating of spores (Mertens *et al.,* 2015; Krogerus *et al.,* 2016) to frequencies as low as 1 × 10⁻⁶ to 1 × 10⁻⁸ for mass mating of cells dependent on rare mating (Gunge and Nakatomi, 1972; Krogerus *et al.,* 2016).

The hybridization frequency of interspecies mating can be improved by genetic modification (GM) techniques, for example by overexpression of the HO endonuclease (Alexander *et al.,* 2016), or by the use of laborious spore micromanipulation (Naumov, 1996). In addition, industrial strains can be resilient to GM, and in general genetically modified organisms (GMO) are not desirable due to low customer acceptance and legislation issues still largely preclude the use of GM technology for applications in the food and beverages industry.

The standard hybrid generation methods used for creation of novel hybrids rely on rather rare events such as loss of heterozygosity at mating type locus (so called rare mating) and require some kind of selection (Krogerus *et al.,* 2015) or spore-to-spore mating techniques using a micromanipulator (Krogerus *et al.,* 2016). The mass mating technique requires a selection to increase the chance of the hybrid appearance and uses a preceding step of sporulation and so meiotic recombination. It increases the uncertainty of the genotype and thus phenotype of the resulting hybrid, and there is this a significant risk of losing or altering desired traits.

### Summary

There is therefore a need for novel and improved methods of generating hybrid yeast strains, and in particular there is a need for efficient methods of generating yeast strains, which are hybrids of *S. eubayanus* and another yeast of the genus *Saccharomyces.*

The present invention provides a novel and highly efficient method of generating hybrid yeast cells. The method relies on the use of a *S. eubayanus* yeast cell comprising a mutation of the HO endonuclease gene, which causes inability of the yeast cell to switch mating type, also termed heterothallic *S. eubayanus.* Such an *S. eubayanus* yeast cell can efficiently mate with another yeast cell of the *Saccharomyces* genus, thus allowing for generation of hybrid yeast cell.

The *S. eubayanus* yeast cell and/or the other yeast cell of the *Saccharomyces* genus may also comprise one or more mutations in other genes. In particular, one or more additional mutations may be introduced into the *S. eubayanus* yeast cells already comprising a mutation of the HO endonuclease genes. Because said *S. eubayanus* yeast cells have an inability to switch mating type, said *S. eubayanus* yeast cells can easily be propagated in a clonal manner. This property facilitates introduction and maintenance of further mutations into the *S. eubayanus* yeast cells.

The invention provides an improved hybridization method that is less labour-intensive and have increased hybridization frequency compared to existing methods.

The invention also relates to a method of producing a beverage using the hybrid yeast cells of the invention.

Thus, the invention provides methods of generating hybrid yeast cells, the method comprising the steps of:
(a) Providing heterothallic *S. eubayanus* yeast cells, wherein said yeast cells comprise a mutation in the gene encoding an HO endonuclease, wherein the wild type HO endonuclease gene comprises the sequence of SEQ. ID. NO. 1 or a sequence sharing at least 80%, such as at least 90%, for example at least 95% sequence identity therewith and which encodes HO endonuclease of SEQ. ID. NO: 2 or a functional homologue thereof; and
(b) mating the *S. eubayanus* yeast of step (a) with yeast of the genus *Saccharomyces,* wherein said *Saccharomyces* has the opposite mating type of the *S. eubayanus* yeast;
thereby obtaining a hybrid yeast cell.

The invention also provides *S. eubayanus* yeast cells, preferably heterothallic *S. eubayanus* yeast cells, wherein said yeast cells comprise a mutation in the gene encoding an HO endonuclease, wherein the wild type HO endonuclease gene comprises the sequence of SEQ. ID. NO. 1 or a sequence sharing at least 80%, such as at least 90%, for example at least 95% sequence identity therewith. The mutation may be any of the mutations described below in the section "HO endonuclease".

The invention also provides hybrid yeasts, for example hybrid yeasts prepared from the *S. eubayanus* yeast cells comprising a mutation in the gene encoding an HO endonuclease and methods of using such hybrid yeasts.

### Description of Drawings

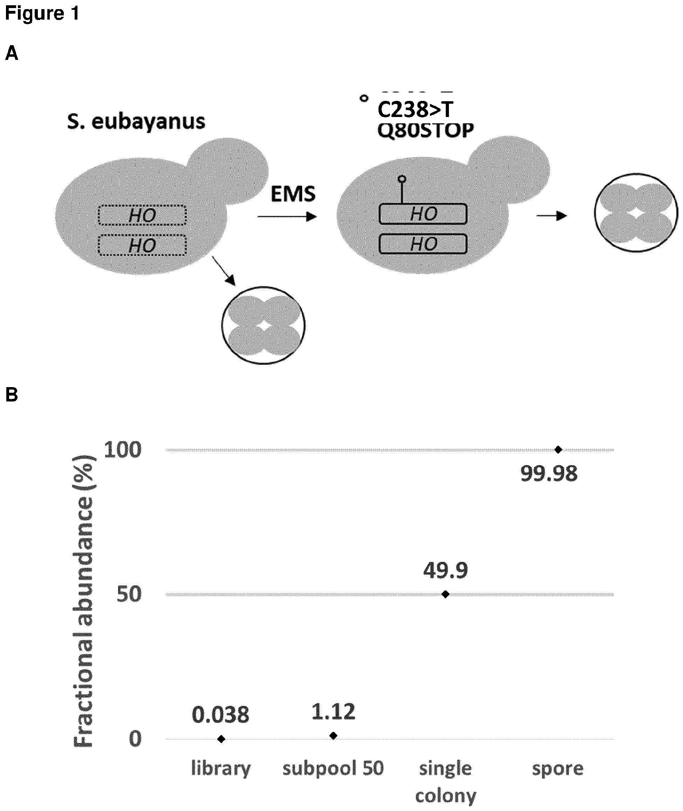

**Figure 1****. (A)** Schematic illustration of the identification of C238T mutation in HO gene in the population of *S*. *eubayanus* strain and isolation of the subsequent mutant spores. **(B)** A graph visualizing the fractional abundance showing the frequency of the mutation in the populations indicated.

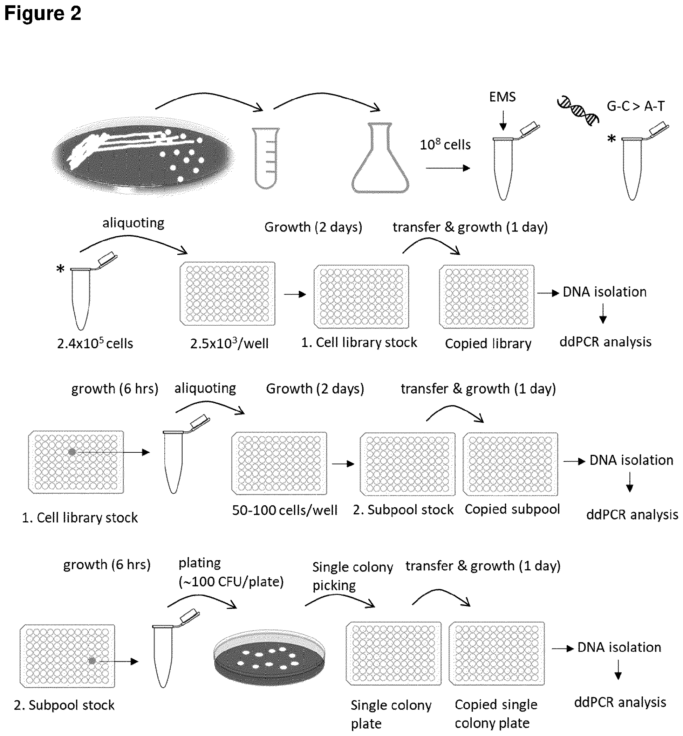

**Figure 2****.** Schematic representation of the experimental procedure for generating yeast cells carrying a specific mutation.

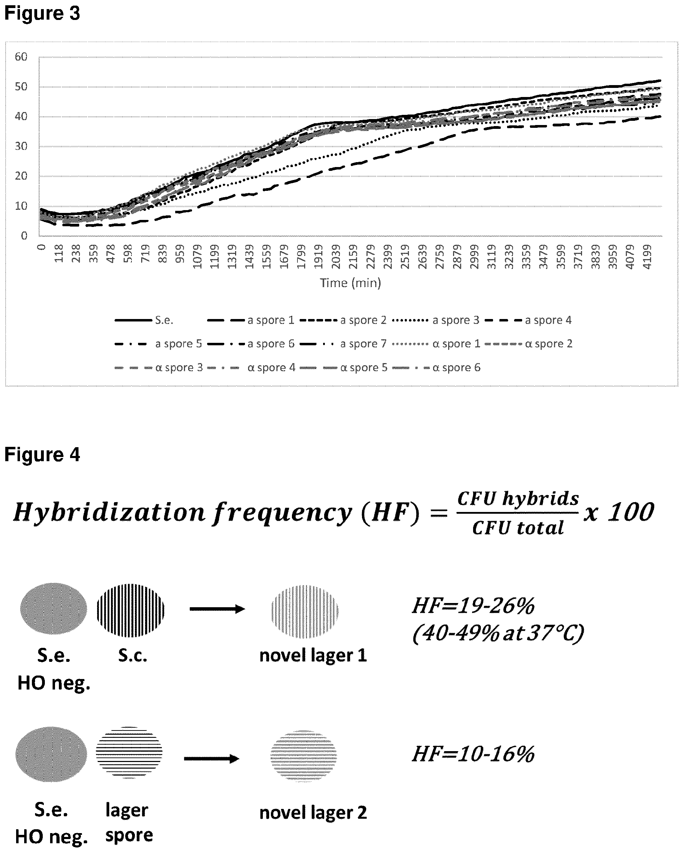

**Figure 3****.** The growth profile of spores from different asci of the hybrids were analyzed and compared with *S. eubayanus.* The values in the graph represent G values (Green value, represents culture density) from the growth profiles over time. There was no significant difference among spores from different asci.
**Figure 4****.** Illustration of the breeding of the HO negative (HO neg.) *S. eubayanus* haploid pure lines with selected *S. cerevisiae* spores. The hybridization frequency (HF) was calculated using the displayed formula. CFU=colony-forming units. HF is given for the two hybrids, novel lager 1 and novel lager 2.

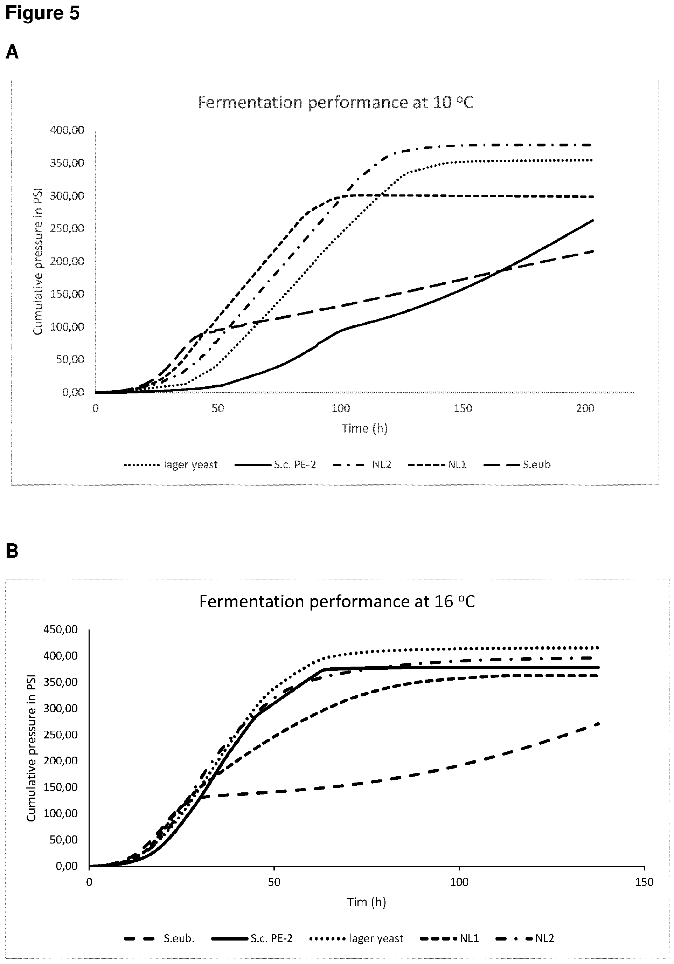

**Figure 5****.** Fermentation profiles of the three parental strains *S. c*. (*S. cerevisiae*)*, S. e.* (*S. eubayanus*) and lager yeast (*S. pastorianus*)*,* as well as the HO negative hybrids novel lager 1 and novel lager 2 are displayed in the chart when fermentation is performed at 10 °C (A) and 16 °C (B). The chart shows the CO₂ production of the strains (as cumulative pressure in PSI). The CO₂ production can be used to estimate the sugar, such as maltose, uptake and fermentation. The graph suggests that the novel lager strains, novel lager 1 and 2, have a better sugar uptake compared to *S. eubayanus.*

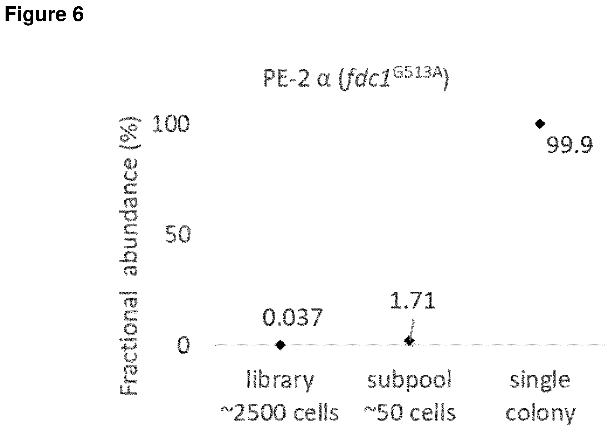

**Figure 6****.** A graph visualizing the fractional abundance showing the frequency of the *fdfc1*^{G513A} mutation in the population of the original library (∼2500 cells), subpool 50 (∼50 cells) and a single colony.

### Detailed description

### Definitions

As used herein, "a" can mean one or more, depending on the context in which it is used.

Amino acids may be named herein using the IUPAC one-letter and three-letter codes. If not otherwise indicated the term "amino acid" refers to the standard amino acids.

The term "aqueous extract" as used herein refers to any aqueous extract of malt and/or cereal kernels. Thus, non-limiting examples hereof can be wort with a given amount of fermentable sugars.

The term "beer" as used herein refers to a beverage prepared by fermentation of wort. Preferably, said fermentation is done by yeast.

The term "cereal" as used herein refers to any plant of the grass family yielding an edible grain, such as wheat, millet, rice, barley, oats, rye, triticale, sorghum, and maize.

The term "completing fermentation" as used herein refers to the point in time during incubation of a yeast in an aqueous extract in a closed container, when said yeast stops consuming sugars of said aqueous extract. Completion of fermentation may be determined by determining the sugar content of the aqueous extract, e.g. by determining the °Plato of the aqueous extract. At the time point when the sugar content does not change significantly, e.g. when the sugar content does not decrease by more than 0.5°Plato over 24h, the fermentation is considered completed. Alternatively, the completion of fermentation may be determined by determining the gas development, e.g. by determining the cumulative pressure within the container. At the time point when the pressure does not change significantly, e.g. when the cumulative pressure does not increase by more than 30 PSI over 24h, the fermentation is considered completed.

The term "corresponding to X" as used herein in relation to amino acids or nucleotides or codons describes amino acids of a given polypeptide or a nucleotide or a codon of a given DNA sequence in relation to amino acids of a reference polypeptide or or nucleotides or codons of a reference DNA sequence. Following alignment between said polypeptide and the reference polypeptide, or said DNA and the reference DNA sequence, an amino acid or nucleotide or codon is corresponding to X if it is in the same position as X in said alignment.

The term "deletions" as used herein may be a deletion of the entire gene, or of only a portion of the gene, or a part of a chromosome.

The term "fermenting" as used herein refers to incubating an aqueous extract with a microorganism, such as a yeast strain. The term "fermentation" refers to such incubation.

The term "functional homologue" as used herein denotes a polypeptide sharing at least one biological function with a reference polypeptide. In general said functional homologue also shares a significant sequence identity with the reference polypeptide, such as at least 60% or at least 70 % or at least 80% or at least 90% or at least 95% or at least 98% sequence identity. Preferably a functional homologue of a reference polypeptide is a polypeptide, which has the same biological function as the reference protein and which shares a high level of sequence identity with the reference polypeptide, such as at least 80% or at least 85%, or at least 90% or at least 95% or at least 98% or at least 99% sequence identity. A high level of sequence identity indicates likelihood that the first sequence is derived from the second sequence.

The term "GMO technique" as used herein refers to a method of inserting foreign genetic material into an organism. Non-limiting examples of GMO techniques are introduction of genetic material into a host cell via vectors, plasmids or phages, resulting in expression of coding sequences directly from these. Alternatively, the introduced coding sequences are integrated into the genome of the host cell and expressed from there. Genome editing techniques, such as CRISPER, can also be used to generate genetically modified organisms (GMO). Cell fusion or hybridisation techniques where live cells with new combinations of heritable genetic material are formed through the fusion of two or more cells by means of methods that do not occur naturally, are also considered GMO techniques.

The term "heterothallic" as used herein refers to a species having sexes that reside in different individuals, which can mate with each other. Accordingly, in heterothallic yeast it requires two compatible partners of opposite mating type for mating to occur. Typically, in heterothallic yeast haploid spores of opposite mating type may mate with each other to form a zygote.

The term "hybridization frequency" as used herein refers to the percentage of hybrid yeast cells obtained after incubation of yeast cells under conditions allowing for hybridisation compared to the total number of colony forming units (CFU). In particular, the "hybridization frequency" may refer to the percentage of diploid hybrids yeast cells obtained after incubation of haploid spores of *S. eubayanus* with haploid spores of another *Saccharomyces* yeast under conditions allowing for hybridisation compared to the total number of CFU. Hybridisation frequency may in particular be determined at room temperature, for example as described in Example 2 below.

The term "malt" as used herein refers to cereal kernels, which have been malted. By the term "malting" is to be understood a process involving steeping and germination of kernels in a process taking place under controlled environmental conditions, optionally followed by a drying step. Said drying step may preferably be kiln drying of the germinated kernels at elevated temperatures. Green malt, which has not been subject to kilning may also be used, in particular malt obtained by the process described in WO 2018/001882. The term "green malt" refers to germinated cereal kernels, which have not been subjected to a step of kiln drying. In some embodiments the green malt is milled green malt. The term "kiln dried malt" as used herein refers germinated cereal kernels, which have been dried by kiln drying. In some embodiments the kiln dried malt is milled kiln dried malt. In general, said cereal kernels have been germinated under controlled environmental conditions.

The term "mutation" as used herein include insertions, deletions, substitutions, transversions, and point mutations in the coding and noncoding regions of a gene. Point mutations may concern changes of one base pair, and may result in premature stop codons, frameshift mutations, mutation of a splice site or amino acid substitutions. A gene comprising a mutation may be referred to as a "mutant gene". If said mutant gene encodes a polypeptide with a sequence different to the wild type, said polypeptide may be referred to as a "mutant polypeptide" and/or "mutant protein". A mutant polypeptide may be described as carrying a mutation, when it comprises an amino acid sequence differing from the wild type sequence.

The term "wild type" in relation to protein or nucleotide sequence refers to a sequence that occurs in nature. There may be some natural occurring variations of protein or nucleotide sequences, however generally there will be one sequence which is the one that is dominantly found within a species, and which is considered to be the wild type sequence in the context of the present application. Generally, the present application will identify the wild type sequence either by a Genebank accession number and/or a SEQ. ID. NO.

The term "sequence identity" as used herein describes the relatedness between two amino acid sequences or between two nucleotide sequences, i.e. a candidate sequence (e.g. a mutant sequence) and a reference sequence (such as a wild type sequence) based on their pairwise alignment. For purposes of the present invention, the sequence identity between two amino acid sequences is determined using the Needleman-Wunsch algorithm (Needleman and Wunsch, 1970, J. Mol. Biol. 48(3): 443-453) as implemented in the Needle program of the EMBOSS package (EMBOSS: The European Molecular Biology Open Software Suite, Rice et al., 2000,Trends Genet. 16: 276-277), preferably version 5.0.0 or later (available at https://www.ebi.ac.uk/Tools/psa/emboss_needle/). The parameters used are gap open penalty of 10, gap extension penalty of 0.5, and the EBLOSUM62 (EMBOSS version of 30 BLOSUM62) substitution matrix. The output of Needle labeled "longest identity" (obtained using the -nobrief option) is used as the percent identity and is calculated as follows: (Identical Residues x 100)/(Length of Alignment - Total Number of Gaps in Alignment). The Needleman-Wunsch algorithm is also used to determine whether a given amino acid in a sequence other than the reference sequence corresponds to a given position of the reference sequence. For purposes of the present invention, the sequence identity between two nucleotide sequences is determined using the Needleman-Wunsch algorithm (Needleman and Wunsch, 1970, supra) as implemented in the Needle program of the EMBOSS package (EMBOSS: The European Molecular Biology Open Software Suite, Rice et al., 2000, Trends Genet. 16: 276-277), preferably version 5.0.0 or later. The parameters used are gap open penalty of 10, gap extension penalty of 0.5, and the DNAFULL (EMBOSS version of NCBI NUC4.4) substitution matrix. The output of Needle labeled "longest identity" (obtained using the -nobrief option) is used as the percent identity and is calculated as follows: (Identical Deoxyribonucleotides x 100)/(Length of Alignment - Total Number of Gaps in Alignment).

The term "stop codon" as used herein refers to a nucleotide triplet in the genetic code, which within mRNA results in termination of translation. The term "stop codon" as used herein also refers to a nucleotide triplet within a gene encoding the stop codon in mRNA. The stop codon in DNA typically has one of the following sequences: TAG, TAA or TGA.

By the term "wort" is meant a liquid extract of malt and/or cereal grains and optionally additional adjuncts. Wort is in general obtained by mashing, optionally followed by "sparging", in a process of extracting residual sugars and other compounds from spent grains after mashing with hot water. Sparging is typically conducted in a lauter tun, a mash filter, or another apparatus to allow separation of the extracted water from spent grains. The wort obtained after mashing is generally referred to as "first wort", while the wort obtained after sparging is generally referred to as the "second wort". If not specified, the term wort may be first wort, second wort, or a combination of both. During conventional beer production, wort is boiled together with hops. Wort without hops, may also be referred to as "sweet wort", whereas wort boiled with hops may be referred to as "boiled wort" or simply as wort.

### Method of generating hybrid yeast cells

As described herein, the present invention relates to a method of generating a hybrid yeast cell, by mating a *S. eubayanus* yeast cell with another yeast cell of the genus *Saccharomyces.* Preferably, said other yeast cell of the genus *Saccharomyces* is not of the species *S. eubayanus.* The invention provides a method that enables generation of yeast hybrids in a fast and less complex way compared to already existing methods. A person skilled in the art will appreciate that the method does not require GMO techniques. Thus, the present invention describes *S. eubayanus* yeast cells useful for generating hybrid yeast cells, which have been generated by non-GMO techniques. In one embodiment the *S. eubayanus* yeast cells of the invention are generated by random mutagenesis and not naturally occurring variants.

In one aspect, the present invention provides a method of generating hybrid yeast cells, the method comprising the steps of:
(a) providing heterothallic *S. eubayanus* yeast cells, wherein said yeast cells comprise a mutation in the gene encoding an HO endonuclease, wherein the wild type HO endonuclease gene comprises the sequence of SEQ. ID. NO. 1 or a sequence sharing at least 80%, such as at least 90%, for example at least 95% sequence identity therewith and which encodes a functional homologue of SEQ. ID. NO: 2; and
(b) mating the *S. eubayanus* yeast of step (a) with yeast of the genus *Saccharomyces,* wherein said *Saccharomyces* has the opposite mating type of the *S. eubayanus* yeast;
thereby obtaining a hybrid yeast cell.

In one embodiment, the functional homologue of SEQ. ID. NO: 2 shares at least 80%, such as at least 90%, for example at least 95% sequence identity therewith, and has the same biological function as SEQ. ID. NO: 2.

It is preferred that spores of said *S. eubayanus* yeast cells and spores of the yeast of the genus *Saccharomyces* are used for mating. Preferably, spores of the S. *eubayanus* yeast of a given mating type, are mated with spores of the other yeast of the genus *Saccharomyces* with the opposite mating type. Thus, if said S. *eubayanus* spores are of mating type a, it is preferred that the spores of the other yeast of the genus *Saccharomyces* are of mating type α and vice versa.

In particular, the mating may involve mating haploid spores of said *S. eubayanus* yeast with spores of said *Saccharomyces* yeast, wherein said spores of *Saccharomyces* has the opposite mating type of the haploid spores of the *S. eubayanus* yeast.

In some embodiments both the spores of said *S. eubayanus* yeast and the spores of said *Saccharomyces* yeast are haploid. In such cases the resulting hybrid yeast cell may be a diploid hybrid yeast cell.

However, in other embodiments spores of a different ploidy may be used, in which case the resulting hybrid yeast cell may be a polyploid hybrid yeast cell. In some embodiments, the ploidy of the spores is unknown. This may for example be the case, in some embodiments where the *Saccharomyces* yeast is *S. pastorianus.*

Said spores of S. *eubayanus* and the other yeast of the genus *Saccharomyces* may be generated by any useful means, for example by incubation of said yeast in media inducing sporulation. Numerous useful media for sporulation are known to the skilled person. In this regard reference is e.g. made to Lundblad and Struhl, 2008.

Spores may be isolated, e.g. with the aid of a dissection microscope, and the spores may be propagated before mating.

The mating type of the spores may be determined by any conventional means, e.g. using a halo assay on a lawn of a mating pheromone sensitive yeast (see e.g. paragraph 2.5 of Kempf et al., 2017 Microbiological Research 200:53-63).

Once spores are propagated, spores of opposite mating type of S. *eubayanus* and the other yeast of the genus *Saccharomyces* may be mated with each other. The mating may be done by any conventional means. Typically, the spores are incubated with each other either in medium supporting growth of yeast or on a solid or semi-solid medium supporting growth of yeast. Said spores are generally supplied in roughly equal amounts. The spores are incubated together for a suitable amount of time, e.g. for in the range of 6 to 24 hours, such as for 10 to 14 hours. Suitable methods for mating of yeast cells are e.g. described in Treco and Winston, 2008.

One advantage of the methods of the invention is the high hybridization frequency. Thus, it is preferred that the hybridization frequency of the S. *eubayanus* yeast strain with the other yeast of the genus *Saccharomyces* is high. Preferably, the hybridization frequency is at least 8%, such as at least 10%, such as at least 15%, such as at least 17%, such as at least 19%, such as at least 21%, such as at least 23%. such as at least 25%.

In particular, it is preferred that the hybridisation frequency of the S. *eubayanus* yeast strain with *S. cerevisiae* yeast is at least 17%, such as at least 19%. Thus, the hybridisation frequency of hybridisation between haploid S. *eubayanus* spores with haploid *S. cerevisiae* spores may be at least 17%, such as at least 19%. Aforementioned hybridisation frequency may in particular be obtained, when hybridization is performed at room temperature..

It is also preferred that the hybridisation frequency of the S. *eubayanus* yeast strain with *S. pastorianus* yeast is at least 8%, such as at least 10%. Thus, the hybridisation frequency of hybridisation between haploid S. *eubayanus* spores with *S. pastorianus* spores may be at least 8%, such as at least 10%. Aforementioned hybridisation frequency may in particular be obtained, when hybridization is performed at room temperature.

A person skilled in the art will appreciate that the methods provided by the invention do not rely on the generation of auxotrophic mutants, which is otherwise necessary to obtain high hybridisation frequencies. Generation of auxotrophic mutants is time-and labor-intensive, and can be further complicated by the polyploid and aneuploid nature of many industrially-relevant *Saccharomyces* strains. Thus, in one embodiment neither the S. *eubayanus* yeast strain nor the other *Saccharomyces* yeast strain have been manipulated to become auxotrophic.

Interestingly, the invention shows that such hybrid yeast cells may have improved properties compared to said *S. eubayanus* yeast cells provided in step (a) and/or compared to the yeast of the genus *Saccharomyces* of step (b). In order, to obtain hybrid yeast cells with desired properties, the methods may further comprise a step of selecting hybrid yeast cells having one or more desired property.

Thus, in one embodiment, the method further comprises a step of selecting hybrid yeast cells, which are capable of producing increased levels of one or more esters compared with the *S. eubayanus* and/or the other yeast of the genus *Saccharomyces* as provided in step (a) and (b) of the method, respectively. The yeasts in step (a) and (b) can also be termed parental yeasts. Said esters may for example be selected from the group consisting of acetate esters and butanoate ester. For example the ester(s) may be acetate esters. For example the esters may be selected from the group consisting of ethylacetate, ethylbutyrate and 2-phenylethyl acetate. Whether a hybrid yeast cell is capable of producing increased levels of an ester may be determined by incubating said hybrid yeast cell in an aqueous extract of malt and/or cereal kernels, such as a wort, and determine the level of ester produced and compare said level of ester to the level of ester produced by the reference yeast when incubated in a similar aqueous extract under the same conditions. Said increased levels may for example be an increase of 10%, such as an increase of 20% of one or more of said esters compared to the level produced by either the *S. eubayanus* and/or the other yeast of the genus *Saccharomyces* as provided in step (a) and (b).

In one embodiment, the method further comprises a step of selecting hybrid yeast cells, which are capable of fermenting maltose. The skilled person is able to determine whether a yeast cell is capable of fermenting maltose. Typically, this may be done by incubating said hybrid yeast cell in a composition comprising maltose (e.g. in an aqueous extract of malt and/or cereal kernels) and determine whether maltose is consumed and/or determine whether fermentation products are increased. Said fermentation product may e.g. be CO₂, the production of which can be determined indirectly by determining pressure if the incubation is performed in a closed container.

In one embodiment, the method further comprises a step of selecting hybrid yeast cells which have an increased real degree of fermentation (RDF) compared to the *S. eubayanus* yeast strain provided in step (a) of the method. RDF may for example be determined as described herein below in the section "Analysis of flavor profiles in fermented wort samples" in the Examples. Said increase may for example be an increase of at least 10%, for example of at least 20%, such as of at least 30%.

In one embodiment, the method further comprises a step of selecting hybrid yeast cells, which have a RDF of at least 60%, such as at least 69%.

In one embodiment, the method further comprises a step of selecting hybrid yeast cells, which have an increased production of alcohol compared to the S. *eubayanus* yeast strain provided in step (a) of the method. Said increased production of alcohol may in particular be obtained after fermenting an aqueous extract of malt and/or cereal kernels with the hybrid yeast. The alcohol production may for example be determined as described herein below in the section "Analysis of flavor profiles in fermented wort samples" in the Examples. Said increase may for example be an increase of at least 10%, for example of at least 15%.

In one embodiment, the method further comprises a step of selecting hybrid yeast cells, which are capable of producing at least 5.0 vol%, such as at least 5.75 vol%, such as at least 6.25 vol%, such as at least 6.75 vol%, such as at least 6.95 vol% alcohol, when incubated with an aqueous extract of malt and/or cereal kernels.

In one embodiment, the method further comprises a step of selecting hybrid yeast cells, wherein the yeast cells comprise a mutation in the gene encoding an HO endonuclease, wherein said mutation may be any of the mutations described herein below in the section "HO endonuclease".

### S. eubayanus yeast strain

The present invention relates to highly efficient methods for creating hybrid yeast cells from an *S. eubayanus* yeast strain and another yeast of the genus *Saccharomyces.* The methods rely on use of an *S. eubayanus* yeast strain carrying a mutation in the HO endonuclease gene. Preferably, said mutation causes a loss-of-function of HO endonuclease as described below in the section "HO endonuclease". Preferably, said mutation in the HO endonuclease causes inability of the *S. eubayanus* yeast cell to switch mating type. For example, it may be determined whether a test yeast strain is able to switch mating type by a method comprising the steps of:
- generating spores of said test yeast strain
- determining mating type of at least one spore
- growing said at least one spore
- testing whether progeny of said at least one spore has the same mating type
wherein the test yeast strain is not able to switch mating type if the progeny of said at least one spore has the same mating type.

It is preferred that said *S. eubayanus* yeast strain is heterothallic.

Thus, the *S. eubayanus* yeast strain of the present invention preferably is unable to switch mating type. This allows maintenance and expansion of the *S. eubayanus* yeast strain in a clonal manner, which again allows to control of the genotype of the *S. eubayanus* yeast strain.

For example, one or more additional mutations may be introduced into the *S. eubayanus* yeast strain, and said additionally mutated *S. eubayanus* yeast strain may be used for mating and production of a hybrid yeast strain. In that manner, specific mutations may be introduced into a hybrid yeast in a controlled manner. Said additional mutation(s) may be any mutations, for example any of the mutations described in the section "Other mutations" herein below.

The *S. eubayanus* yeast may be any *S. eubayanus* available to the skilled person. Typically, *S. eubayanus* is a cryotolerant type of yeast capable of fermenting glucose at reduced temperatures (e.g. at temperatures in the range of 8 to 15°C, such as at 10°C). *S. eubayanus* may also be able to ferment maltose, albeit in general with less efficiency than *S. pastorianus.*

The skilled person will be able to determine whether a given yeast belongs to the species *S. eubayanus,* for example based on the genomic sequence of the yeast. As reference the sequence of the genomic sequence of *S. eubayanus* described by Baker *et al.* 2015 may be used.

The methods of the invention comprise a step of mating. Preferably, said step comprises mating spores of the S. *eubayanus* yeast of a given mating type, with spores another yeast of the genus *Saccharomyces* with the opposite mating type. Thus, said S. *eubayanus* spores may for example be of mating type a or of mating type α.

### Saccharomyces yeast strain

The present invention relates to highly efficient methods for creating hybrid yeast cells from an *S. eubayanus* yeast strain and another yeast of the genus *Saccharomyces.* Said other yeast of the genus *Saccharomyces* may be any yeast of the genus *Saccharomyces,* however it is preferred that the other yeast is not an *S. eubayanus* yeast strain.

Whereas the other yeast of the genus *Saccharomyces* may be able to switch mating types, it is preferred that said other yeast is heterothallic.

In one embodiment of the present invention, the other yeast of the genus *Saccharomyces* is selected from the group consisting of *S. cerevisiae, S. pastorianus, S. kudriavzevii, S. bayanus, S. uvarum, Saccharomyces cariocanus* and *Saccharomyces mikatae.*

In one embodiment of the present invention, the other yeast of the genus *Saccharomyces* is selected from *S. cerevisiae or S. pastorianus.*

In a preferred embodiment of the invention, the other yeast of the genus *Saccharomyces* is *S. cerevisiae.* In such embodiments, the hybrid yeast will be a hybrid of *S. eubayanus* and *S. cerevisiae,* and thus said hybrid may be a yeast of the species *S. pastorianus.*

The methods of the invention comprise a step of mating. Preferably, said step comprises mating spores of the S. *eubayanus* yeast of a given mating type, with spores another yeast of the genus *Saccharomyces* with the opposite mating type. Thus, if said S. *eubayanus* spores of mating type a, it is preferred that the spores of the other yeast of the genus *Saccharomyces* are of mating type α and vice versa.

### HO endonuclease

In one aspect, the present invention provides a *S. eubayanus* yeast cell, wherein said yeast cell comprises a mutation in the gene encoding an HO endonuclease. The invention further provides hybrids of said *S. eubayanus* yeast cell and another yeast of the genus *Saccharomyces,* and thus said hybrids in general also comprise a mutation in the gene encoding an HO endonuclease.

The gene encoding an HO endonuclease is also referred to as "HO endonuclease gene" herein.

The wild type sequence of the HO endonuclease gene of *S. eubayanus* is provided herein as SEQ. ID. NO: 1. As will be appreciated by the skilled person, the HO endonuclease gene of various *S. eubayanus* strains may have slight differences, and thus the wild type HO endonuclease gene may comprise or consist of a sequence of SEQ. ID. NO. 1 or a sequence with at least 80%, such as at least 90%, for example at least 95%, such as at least 98%, such as at least 99% sequence identity with SEQ. ID. NO: 1. In particular, it is preferred that the wild type HO endonuclease gene encodes HO endonuclease of SEQ. ID. NO:2 or a functional homologue thereof sharing at least 80%, such as at least 90%, for example at least 95% such as at least 98%, such as at least 99% sequence identity with SEQ. ID. NO: 2.

In one embodiment, the mutation in the HO endonuclease causes inability of the *S. eubayanus* yeast cell to switch mating type. Thus, it is preferred that the mutation causes a loss-of-function of HO endonuclease.

In one embodiment, the mutation is point mutation. In one embodiment the mutation is a frameshift mutation. In one embodiment, the mutation occurs in the coding region. In one embodiment, the mutation introduces a premature stop codon in the coding region.

In particular, it is preferred that the mutation in the HO gene results in a mutated gene encoding a mutant HO endonuclease. Preferably, said mutant HO endonuclease has a total loss-of-function. In particular, said mutant HO endonuclease may be truncated, i.e. it may lack part of the wild type HO endonuclease. Frequently, said mutant HO endonuclease comprise the N-terminal part of HO endonuclease, but lacks the C-terminal part. This may for example be the case, if the mutation introduces a pre-mature stop codon or a frame shift.

Thus, in one embodiment, the mutated HO endonuclease gene encodes a mutant HO endonuclease lacking at least the 100 most C-terminal amino acids, such as lacking at least the 200 most C-terminal amino acids, for example lacking at least the 300 most C-terminal amino acids, such as lacking at least the 400 most C-terminal amino acids, preferably lacking at least the 510 most C-terminal amino acids of SEQ. ID. NO: 2.

In one embodiment, the mutation introduces a premature stop codon in a codon corresponding to any one of codons 1 to 500, such as in any one of codons 1 to 400, for example in any one of codons 1 to 300, such as in any one of codons 1 to 200, preferably in any one of codons 1 to 80 of SEQ. ID. NO: 1.

In one embodiment, the mutation is a mutation from C to T at the position corresponding to nucleotide 238 of SEQ. ID. NO. 1.

In one embodiment, the mutant HO endonuclease coding sequence comprises or consists of the sequence of SEQ. ID. NO: 3.

In one embodiment, the mutated HO endonuclease coding sequence encodes a mutant HO endonuclease carrying a Gln to stop mutation at the amino acid at the position corresponding to amino acid 80 of SEQ. ID. NO:2.

The mutation in the HO endonuclease gene may be introduced into *S. eubayanus* by any suitable means. Preferably, the *S. eubayanus* strain of the invention has been generated by non-GMO methods. In one embodiment the *S. eubayanus* yeast cells of the invention are generated by random mutagenesis by exposing the cells or spores to a mutagen, such as UV radiation or a mutagenic chemical such as ethyl methanesulfonate. One preferred method for producing a *S. eubayanus* strain according to the invention is the method described in international patent application WO 2018/001884. Said method allow for identification of any desirable, specific mutation in yeast by non-GMO means. Preferably, the *S. eubayanus* strain carrying the mutation in the HO endonuclease gene is identified using the method schematically outlined in figure 2. A more specific method for producing a *S. eubayanus* strain carrying the mutation in the HO endonuclease gene is described below in Example 1.

### Other mutations

As described herein, the present invention relates to an *S. eubayanus* yeast strain carrying a mutation in the HO endonuclease gene. In addition to said mutation, the *S. eubayanus* yeast strain may carry one or more additional mutations in one or more genes of interest. The invention also provides methods of producing hybrid yeast strains by mating said *S. eubayanus* yeast strain with another yeast of the genus *Saccharomyces.* Said other yeast of the genus *Saccharomyces* may also carry one or more additional mutations. It follows that the hybrid yeast strains produced by the methods of the invention also may carry said one or more additional mutations.

The additional mutation(s) may be any mutations introducing a trait of interest. Numerous mutations are known to be associated with a desirable phenotype, and thus the additional mutation may be any such mutation.

As an example of an additional useful mutation, the invention provides *S. eubayanus* yeast cells as well as other yeast of the genus *Saccharomyces* comprising a mutation in the FDC1-like gene. Inactivation of *FDC1* in *S. cerevisiae* or *S. pastorianus* is known to abolish the production of 4-VG, resulting in a yeast strain without phenolic off-flavors (POF negative).

Thus, the *S. eubayanus* yeast cells and/or the other yeast of the genus *Saccharomyces,* may comprise a mutation in the gene encoding FDC1-like. The wild type sequence of the FDC1-like gene of *S. eubayanus* is provided herein as SEQ. ID. NO: 4 and of *S. cerevisiae* as SEQ. ID. NO:6. As will be appreciated by the skilled person, the FDC1-like gene of various yeast strains may have slight differences, and thus the wild type FDC1-like gene may comprise or even consist of a sequence of SEQ. ID. NO. 4 or SEQ. ID. NO:6 or a sequence sharing at least 80%, such as at least 90%, for example at least 95% sequence identity therewith. In particular, it is preferred that the wild type FDC1-like gene encodes FDC1-like of SEQ. ID. NO:5 or SEQ. ID. NO:7 or a functional homologue thereof sharing at least 80%, such as at least 90%, for example at least 95% sequence identity therewith.

In one embodiment, the mutated *FDC1-like* gene encodes a mutant FDC1-like polypeptide lacking at least the 100 most C-terminal amino acids, such as lacking at least the 200 most C-terminal amino acids, for example lacking at least the 300 most C-terminal amino acids, preferably lacking at least the 333 most C-terminal amino acids of SEQ. ID. NO: 5 or SEQ. ID. NO:7.

In one embodiment, the mutation in the *FDC1-like* gene introduces a premature stop codon in a codon corresponding to any one of codons 1 to 400, for example in any one of codons 1 to 300, such as in any one of codons 1 to 200, preferably in any one of codons 1 to 171 of SEQ. ID. NO: 4 or SEQ. ID. NO:6.

The mutation in the additional gene may be introduced into *S. eubayanus* or the other yeast of the genus *Saccharomyces* by any suitable means, preferably by non-GMO methods. In one embodiment the *S. eubayanus* yeast cells of the invention are generated by random mutagenesis by exposing the cells or spores to a mutagen, such as UV radiation or a mutagenic chemical such as ethyl methanesulfonate. One preferred method for producing a yeast strain carrying a specific mutation is the method described in international patent application WO 2018/001884. Preferably, the *S. eubayanus* strain and/or the other Saccharomyces yeast carrying the additional mutation using the method schematically outlined in Figure 2. A more specific method is described below in Example 4. The skilled person will appreciate that other mutations may be identified by said method by using primers and probes designed for identifying said other mutation.

### The hybrid yeast strain

As disclosed herein, the present invention relates to methods for generating hybrid yeast strains.

Thus, in one aspect, the present invention provides a hybrid yeast strain obtained by the method of generating hybrid yeast strains as described herein. The hybrid yeast cell may be a hybrid of an *S. eubayanus* yeast cell comprising a mutation in the gene encoding an HO endonuclease as described above and another yeast of the genus *Saccharomyces.*

In one aspect, the present invention provides a hybrid yeast strain comprising a mutation in the gene encoding an HO endonuclease, wherein hybrid yeast cell is a hybrid of an *S. eubayanus* yeast cell and a *Saccharomyces* yeast cell. Said mutation may for example be any of the mutations described herein above in the section "HO endonuclease".

In one preferred embodiment of the invention, the hybrid yeast cell has a loss of function mutation in the HO endonuclease gene on at least two alleles, preferably on all alleles. For example, both the *S. eubayanus* yeast and the other yeast of the genus *Saccharomyces* used for generation of the hybrid yeast may both comprise a mutation in the gene encoding HO endonuclease, preferably both yeasts may comprise a loss of function mutation in the gene encoding HO endonuclease. In such cases, the hybrid yeast may have a complete loss-of-function of HO endonuclease due to said mutations. Said mutations in the HO endonuclease gene of the *S. eubayanus* yeast and the other yeast of the genus *Saccharomyces* used for generation of the hybrid may be the same or different mutations, e.g. any of the mutations described in the section "HO enconuclease"..

In one embodiment, the hybrid yeast is of the species *S. pastorianus. S. pastorianus* is used in the production of lager beer. Hallmarks of *S. pastorianus* includes that it is
- bottom fermenting, e.g. it has high flocculation
- cold tolerant
- capable of utilising various sugars, for example melibiose and/or maltotriose

Thus, in one embodiment of the invention, the hybrid yeast strain has high flocculation. Flocculation may for example be determined as cells in solution after fermentation. "Cells in solution" is in general determined by counting the yeast cells in a sample taken from the upper half of the fermentation container. A low number of cells, e.g. at the most 10, preferably at the most 2, even more preferably at the most 1 million cells per millilitre, in solution after fermentation is indicative of high flocculation.

In one embodiment, the hybrid yeast strain is cold tolerant. Preferably, the hybrid yeast cell is capable of fermenting glucose and/or maltose at a temperature in the range of 8 to 15°C, such as at 10°C. In particular, it is preferred that the yeast cell is capable of completing fermentation within 6 days, for example within 130 hours when incubated in an aqueous extract of malt and/or cereal kernels at a temperature in the range of 8 to 15°C, such as at 10°C.

In one embodiment of the present invention, the hybrid yeast strain is capable of utilizing melibiose as sole carbon source. Thus, it is preferred that the yeast strain is capable of growing in an aqueous solution containing melibiose as the sole carbon source.

In one embodiment of the present invention, the hybrid yeast strain is capable utilizing maltotriose as sole carbon source. Thus, it is preferred that the yeast strain is capable of growing in an aqueous solution containing maltotriose as the sole carbon source.

Such aqueous solution preferably do not contain any monosaccharides, disaccharides, trisaccharides and/or tetrasaccharides apart from either melibiose or maltotriose, and more preferably such aqueous solution does not contain any carbohydrates apart from either melibiose or maltotriose.

In one embodiment, the hybrid yeast strain has one or more of the properties, which are described as properties selected for in the section "Method of generating hybrid yeast cells". For example the hybrid yeast strain may have one or more of the following properties:
- capable of producing increased levels of one or more esters compared with the parental *S. eubayanus* and/or the other yeast of the genus *Saccharomyces*
- capable of fermenting maltose
- have an increased real degree of fermentation (RDF) compared to the parent *S. eubayanus* yeast strain
- have an RDF of at least 60%, such as at least 69%
- increased production of alcohol compared to the parent *S*. *eubayanus* yeast strain
- capable of producing at least 5.0 vol%, such as at least 5.75 vol%, such as at least 6.25 vol%, such as at least 6.75 vol%, such as at least 6.95 vol% alcohol, when incubated with an aqueous extract of malt and/or cereal kernels
wherein said properties may be determined as described above in the section "Method of generating hybrid yeast cells".

In addition to the mutation in the HO endonuclease gene said hybrid yeast cell may comprise one or more additional mutations, such as any of the mutations described herein above in the section "Other mutations".

### Method of preparing a beverage and a beverage as such

The hybrid yeast cell described above may be used in the production of a beverage, for example a beverage selected from the group consisting of beer, cider and wine.

In one aspect, the invention provides a method of preparing a beverage, the method comprising the steps of:
(a) Providing an aqueous extract of malt and/or cereal kernels;
(b) providing a hybrid yeast cell as described herein;
(c) fermenting said aqueous extract with said yeast;
thereby obtaining said malt and/or cereal-based beverage.

The aqueous extract may be prepared by incubating malt and/or cereal kernels, which may have been milled, with water. Preferably, the aqueous extract is wort. In particular, the aqueous extract may be prepared by a process known as mashing. Mashing may be performed in the presence of additional ingredients, e.g. adjuncts in addition to malt and/or cereals. It is also possible that enzymes are added during mashing. Any useful mashing methods may be employed, including, but not limited to such methods described by Briggs *et al.* (1981) or Hough *et al.* (1982).

After mashing the aqueous extract may optionally be heated, e.g. boiled, and one or more additional ingredients, e.g. hops may be added.

The aqueous extract may be fermented with the yeast by any useful method, e.g. by any method conventionally used during brewing of beer. Useful methods are e.g. described in Briggs *et al.* (1981) and Hough *et al.* (1982). Numerous, regularly updated methods for analyses of kernel, malt and beer products are available, for example, but not limited to, American Association of Cereal Chemists (1995), American Society of Brewing Chemists (1992), European Brewery Convention (1998), and Institute of Brewing (1997). It is recognized that many specific procedures are employed for a given brewery, with the most significant variations relating to local consumer preferences. Any such method of producing beer may be used with the present invention.

In one aspect, the invention provides a beverage produced according to the method described above.

In one embodiment, the invention provides a beverage comprising at least 0.04mg/l ethyl butyrate, such as at least 0.05mg/l ethyl butyrate.

In one embodiment, the invention provides a beverage comprising at least 20mg/l isobutanol, such as at least 25mg/l isobutanol.

In one embodiment, the invention provides a beverage comprising at least 80mg/l isoamyl alcohol, such as at least 90mg/l isoamyl alcohol, such as at least 100mg/l isoamyl alcohol, such as at least 110mg/l isoamyl alcohol, such as at least 120mg/l isoamyl alcohol, such as at least 125mg/l isoamyl alcohol.

In one embodiment, the invention provides a beverage comprising at least 60mg/l 2-phenylethanol, such as at least 65mg/l 2-phenylethanol, such as at least 70mg/l 2-phenylethanol.

### Items

The invention may further be defined by the following items:
1. A method of generating hybrid yeast cells, the method comprising the steps of:
   (a) Providing heterothallic *S. eubayanus* yeast cells, wherein said yeast cells comprise a mutation in the gene encoding an HO endonuclease, wherein the wild type HO endonuclease gene comprises the sequence of SEQ. ID. NO. 1 or a sequence sharing at least 80%, such as at least 90%, for example at least 95% sequence identity therewith and which encodes HO endonuclease of SEQ. ID. NO: 2 or a functional homologue thereof; and
   (b) mating the *S. eubayanus* yeast of step (a) with yeast of the genus *Saccharomyces,* wherein said *Saccharomyces* has the opposite mating type of the *S. eubayanus* yeast;
   thereby obtaining a hybrid yeast cell.
2. The method according to item 1, wherein the functional homologue of HO endonuclease of SEQ. ID. NO: 2 shares at least 80%, such as at least 90%, for example at least 95% sequence identity therewith, and has the same biological function as SEQ. ID. NO: 2.
3. The method according to any one of the preceding items, wherein step (b) comprises mating spores of said *S. eubayanus* yeast with spores of said *Saccharomyces* yeast, wherein said spores of *Saccharomyces* has the opposite mating type of the spores of the *S. eubayanus* yeast.
4. The method according to item 3, wherein said spores of said *S. eubayanus* yeast are haploid spores.
5. The method according to any one of items 3 and 4, wherein said spores of said *Saccharomyces* are haploid spores.
6. The method according to item 5, wherein the hybrid yeast cell is a diploid hybrid yeast cell.
7. The method according to any one of the preceding items, wherein the mutation in the HO endonuclease causes inability of the yeast cell to switch mating type.
8. The method according to any one of the preceding items, wherein the yeast of the genus *Saccharomyces* of step (b) is heterothallic.
9. The method according to any one of the preceding items, wherein the yeast of the genus *Saccharomyces* is selected from the group consisting of *S. cerevisiae, S. pastorianus, S. kudriavzevii, S. bayanus, S. uvarum, Saccharomyces cariocanus* and *Saccharomyces mikatae.*
10. The method according to any one of the preceding items, wherein the yeast of the genus *Saccharomyces* is selected from the group consisting of *S. cerevisiae* and *S. pastorianus.*
11. The method according to any one of the preceding items, wherein the method further comprises a step of selecting hybrid yeast cells which are capable of producing increased levels of esters, for example increased levels of one or more esters selected from the group consisting of ethylacetate, ethylbutyrate and 2-phenylethyl acetate, compared with one or both of the parent *S. eubayanus* and *Saccharomyces* strains provided in step (a) and (b), respectively.
12. The method according to any one of the preceding items, wherein the method further comprises a step of selecting hybrid yeast cells, which are capable of fermenting maltose.
13. The method according to any one of the preceding items, wherein the method further comprises a step of selecting hybrid yeast cells which have an increased real degree of fermentation (RDF) compared to the *S. eubayanus* yeast strain provided in step (a).
14. The method according to any one of the preceding items, wherein the method further comprises a step of selecting hybrid yeast cells which have a RDF of at least 60%, such as at least 69%.
15. The method according to any one of the preceding items, wherein the method further comprises a step of selecting hybrid yeast cells, which have an increased production of alcohol compared to the S. *eubayanus* yeast strain provided in step (a).
16. The method according to any one of the preceding items, wherein the method further comprises a step of selecting hybrid yeast cells, which are capable of producing at least 5.0 vol%, such as at least 5.75 vol%, such as at least 6.25 vol%, such as at least 6.75 vol%, such as at least 6.95 vol% alcohol, when incubated with a cereal extract.
17. The method according to any one of the preceding items, wherein the method further comprises a step of selecting hybrid yeast cells, wherein the yeast cells comprise a mutation in the gene encoding an HO endonuclease, wherein the wild type HO endonuclease gene comprises the sequence of SEQ. ID. NO. 1 or a sequence sharing at least 80%, such as at least 90%, for example at least 95% sequence identity therewith.
18. The method according to any one of the preceding items, wherein the hybridization frequency of the S. *eubayanus* yeast strain of step (a) with the yeast of the genus *Saccharomyces* is at least 8%, such as at least 10%.
19. The method according to any one of the preceding items, wherein the hybridization frequency of the S. *eubayanus* yeast strain of step (a) with haploid *S. cerevisiae* yeast spores is at least 17%, for example at least 19%, when hybridization was performed at room temperature.
20. The method according to any one of the preceding items, wherein the hybridization frequency of the S. *eubayanus* yeast strain of step (a) with haploid *S. pastorianus* yeast spores is at least 8%, such as at least 10%, when hybridization was performed at room temperature.
21. The method according to any one of the preceding items, wherein the heterothallic *S. eubayanus* yeast cells further comprises one or more additional mutations in one or more genes of interest.
22. A *S. eubayanus* yeast cell, wherein said yeast cell preferably is a heterothallic *S. eubayanus* yeast cell, and wherein said yeast cell comprises a mutation in the gene encoding an HO endonuclease, wherein the wild type HO endonuclease gene comprises the sequence of SEQ. ID. NO. 1 or a sequence sharing at least 80%, such as at least 90%, for example at least 95% sequence identity therewith.
23. The yeast cell according to item 22, wherein the mutation results in loss of function of the protein encoded by mutated HO endonuclease gene.
24. The yeast cell according to item 22 or 23, wherein the mutation is a point mutation.
25. The yeast cell according to any one of items 22 to 24, wherein the mutation is a frameshift mutation.
26. The yeast cell according to any one of items 22 to 25, wherein the mutation occurs in the coding region.
27. The yeast cell according to any one of items 22 to 26, wherein the mutation introduces a premature stop codon in the coding region.
28. The yeast cell according to any one of items 22 to 27, wherein the mutated HO endonuclease gene encodes a mutant HO endonuclease lacking at least the 100 most C-terminal amino acids, such as lacking at least the 200 most C-terminal amino acids, for example lacking at least the 300 most C-terminal amino acids, such as lacking at least the 400 most C-terminal amino acids, preferably lacking at least the 510 most C-terminal amino acids of SEQ. ID. NO: 2.
29. The yeast cell according to any one of items 22 to 28, wherein the mutation introduces a premature stop codon in a codon corresponding to any one of codons 1 to 500, such as in any one of codons 1 to 400, for example in any one of codons 1 to 300, such as in any one of codons 1 to 200, preferably in any one of codons 1 to 80 of SEQ. ID. NO: 1.
30. The yeast cell according to any one of items 22 to 29, wherein the mutation is a mutation from C to T at the position corresponding to nucleotide 238 of SEQ. ID. NO. 1.
31. The yeast cell according to any one of items 22 to 30, wherein the mutated HO endonuclease gene encodes a mutant HO endonuclease carrying a Gln to stop mutation at the amino acid position 80 of SEQ. ID. NO:2.
32. The yeast cell according to any one of items 22 to 31, wherein said heterothallic *S. eubayanus* yeast cells further comprises a mutation in the FDC1-like gene, wherein the FDC1-like gene comprises the sequence of SEQ. ID. NO. 4 or a sequence sharing at least 80%, such as at least 90%, for example at least 95% sequence identity therewith.
33. The yeast cell according to items 32, wherein the mutated *FDC1-like* gene encodes a mutant FDC1-like polypeptide lacking at least the 100 most C-terminal amino acids, such as lacking at least the 200 most C-terminal amino acids, for example lacking at least the 300 most C-terminal amino acids, preferably lacking at least the 333 most C-terminal amino acids of SEQ. ID. NO: 5.
34. The yeast cell according to any one of items 32 to 33, wherein the mutation in the *FDC1-like* gene introduces a premature stop codon in a codon corresponding to any one of codons 1 to 400, for example in any one of codons 1 to 300, such as in any one of codons 1 to 200, preferably in any one of codons 1 to 171 of SEQ. ID. NO: 4.
35. A hybrid yeast cell obtained by the method according to any one of items 1 to 21.
36. A hybrid yeast cell comprising a mutation in the gene encoding an HO endonuclease, wherein the wild type HO endonuclease gene comprise the sequence of SEQ. ID. NO. 1 or a sequence sharing at least 80%, such as at least 90%, for example at least 95% sequence identity therewith, wherein the hybrid yeast cell is a hybrid of an *S. eubayanus* yeast cell and a *Saccharomyces* yeast cell.
37. The hybrid yeast cell according to any one of items 35 to 36, wherein the hybrid yeast cell has a loss of function mutation in the HO endonuclease gene on at least two alleles, preferably on all allelles.
38. The hybrid yeast cell according to item 35 or 37, wherein the *S. eubayanus* yeast cell is the *S. eubayanus* according to any one of items 22 to 34.
39. The hybrid yeast cell according to any one of items 35 to 38, wherein the hybrid yeast is of the species *S. pastorianus.*
40. The hybrid yeast cell according to any one of items 35 to 39, wherein the yeast cell comprises one or more mutations defined in any one of items 22 to 34.
41. The hybrid yeast cell according to any one of items 35 to 39, wherein the hybrid yeast cell the property selected for according to any one of items 11 to 16.
42. The hybrid yeast cell according to any one of items 35 to 41, wherein the yeast cell is capable of fermenting maltose at a temperature in the range of 8 to 15°C, such as at 10°C.
43. The hybrid yeast cell according to any one of items 35 to 42, wherein the yeast cell is capable of completing fermentation within 6 days, for example within 130 hours when incubated with an aqueous extract of malt and/or cereal kernels at a temperature in the range of 8 to 15°C, such as at 10°C.
44. Use of the hybrid yeast cells obtained by the method according to any one of items 1 to 21, and/or the hybrid yeast cell according to any one of items 35 to 43, for the production of a beverage, for example a beverage selected from the group consisting of beer, cider and wine.
45. A method of preparing a beverage, the method comprising the steps of:
   (a) Providing an aqueous extract of malt and/or cereal kernels;
   (b) providing the hybrid yeast cells obtained by the method according to any one of items 1 to 21, and/or the hybrid yeast cell according to any one of items 35 to 43;
   (c) fermenting said aqueous extract with said yeast;
   thereby obtaining said malt and/or cereal-based beverage.
46. The method according to item 45, wherein the aqueous extract is wort or a fermented malt and/or cereal-based beverage.
47. A beverage produced according to the method according to any one of items 45 to 46.
48. The beverage according to item 47, wherein the beverages comprises at least 0.04mg/l ethyl butyrate, such as at least 0.05mg/l ethyl butyrate.
49. The beverage according to any one of items 47 to 48, wherein the beverage comprises at least 20mg/l isobutanol, such as at least 25mg/l isobutanol.
50. The beverage according to any one of items 47 to 49, wherein the beverage comprises at least 80mg/l isoamyl alcohol, such as at least 90mg/l isoamyl alcohol, such as at least 100mg/l isoamyl alcohol, such as at least 110mg/l isoamyl alcohol, such as at least 120mg/l isoamyl alcohol, such as at least 125mg/l isoamyl alcohol.
51. The beverage according to any one of items 47 to 50, wherein the beverage comprises at least 60mg/l 2-phenylethanol, such as at least 65mg/l 2-phenylethanol, such as at least 70mg/l 2-phenylethanol.

### Examples

### Materials and Methods

### Yeast growth medium

All yeast strains used in the example are listed in Table 1. Strains were grown in standard yeast extract peptone dextrose (YPD medium) (1% yeast extract, % peptone, 2% glucose) unless otherwise indicated below at various temperatures based on a particular purpose. For solid media 2% agar was added.

### Sporulation medium

Solid sporulation medium contained 1% potassium acetate, 0.1 % yeast extract, 0.1% glucose and 2% agar.

### Selection plates for S. eubayanus or lager spore derived hybrid

Yeast strains with a functional MEL1 gene encodes an enzyme with alpha-galactosidase activity. MEL1 is regulated by the GAL genes which are triggered by the presence of galactose. Alpha-galactosidase converts the colourless X-gal to the blue product.
MEL1 is in *S. eubayanus,* but often absent in wine or brewer's *cerevisiae.* Therefore galactose-X-gal plates can be used to detect hybrids with *S. eubayanus* origin.

For identification of *S. eubayanus* colonies or *S. eubayanus* hybrid colonies, glucose was replaced with 2% galactose (YPG) and X-gal (5 mg/ml in dimethylformamide) spread in 200 µl on agar plates.

For selection of lager yeast spore-derived hybrids, SG medium (0,67% yeast nitrogen base without amino acids, 2% galactose) supplemented with sulfometuron methyl (in the final concentration 10 mg/ml; SG plates) and X-gal added as described above was used.

### Wort

For fermentation a wort prepared from grist made of 70% Pilsner malt and 30% barley and mashed in 2.7 L water/kg. The mashing profile was: mashing in at 50°C and held for 30 minutes, then raised to 65°C and held for 50 min, the mash was further raised to 78°C and held for 5 min. The extract concentration in the final wort used in the present experiments was 16° Plato.

**Table 1. Yeast strains used or developed in the examples.**

| **Strain name** | **Organism** | **Description** | **Genotype** | **Source** |
|---|---|---|---|---|
| **Parental strains** | | | | |
| S.c. PE-2 | *S. cerevisiae* | bioethanol strain | *MATa*/*MATα Scho* | Basso *et al.,* 2008 And Argueso *et al.,* 2009 |
| Lager yeast | *S. pastorianus* | bottom-fermenting yeast (*S. cerevisiae* x *S eubayanus*) | *unknown* | |
| S.e. CBS12357 | *S. eubayanus* | wild yeast | *MATa*/*MATα* | Libkind *et al.,* 2011 |

| **Strains generated in the Examples** | | | | |
|---|---|---|---|---|
| S.e. ho/HO | *S. eubayanus* | mutagenized *S. eubayanus* strain | *MATa*/*MATα SeHO*/*Seho^{C238T}* | |
| S.c. PE-2 a | *S. cerevisiae* | S.c. PE-2 spore | *MATa Scho* | |
| S.c. PE-2 α | *S. cerevisiae* | S.c. PE-2 spore | *MATα Scho* | |
| S.c. PE-2 a fdc1 | *S. cerevisiae* | mutagenized PE-2 POF negative spore | *MATa fdc1^{G513A}* | |
| S.c. PE-2 α fdc1 | *S. cerevisiae* | mutagenized PE-2 POF negative spore | *MATα fdc1^{G513A}* | |
| lager spore a | *S. pastorianus* | *S. pastorianus* spore clone | *MATa*/*?* | |
| S.e. ho a | *S. eubayanus* | HO negative S.e. spore | *MATa Seho^{C238T}* | |
| S.e. ho α | *S. eubayanus* | HO negative S.e. spore | *MATα Seho^{C238T}* | |
| S.e. fdc1 a | *S. eubayanus* | mutagenized S.e. ho a POF negative spore | *MATa Seho^{C238T} Sefdc1^{G477A}* | |
| S.e. fdc1 α | *S. eubayanus* | mutagenized S.e. ho α POF negative spore | *MATα Seho^{C238T} Sefdc1^{G477A}* | |
| Novel lager 1 (NL1) | *S. cerevisiae x S. eubayanus* | hybrid of S.c. PE-2 a and S.e. ho α | *MATa*/*MATα Seho^{C238T} Seho^{C238T}* | |
| Novel lager 2 (NL2) | *S. pastorianus spore x S. eubayanus* | hybrid of lager spore a and S.e. ho α | *MAT unknown, behaves like MATaSeho^{C238T}* | |
| Novel lager 3 (NL3) | *S. cerevisiae x S. eubayanus* | hybrid of S.c. PE-2 a fdc1 and S.e. fdc1 α | *MATa*/*MATα Scfdc1^{G513A} Seho^{C238T} Sefdc1^{G477A}* | |
| Novel lager 4 (NL4) | *S. pastorianus spore x S. eubayanus* | hybrid of lager spore a and S.e. ho α | *MAT unknown, behaves like MATa Seho^{C238T} Sefdc1^{G477A}* | |

| ***Assay related strains*** | | | | |
|---|---|---|---|---|
| α-PS | *S. cerevisiae* | S.c. BY4741 α pheromone sensitive strain | *MATa sst2D* | Euroscarf |
| a-PS | *S. cerevisiae* | S.c. BY4742 a pheromone sensitive strain | *MATα sst2D* | Euroscarf |

### Yeast growth conditions

In microscale the yeast strains were grown as starter cultures in Greiner 96-well microtiter plates in 150 µl of YPD. 10 µl of the overnight cultures were transferred into 96-well square deep-well plates filled with 240 µl of wort. The sporulation plates were covered with anaerobic sandwich covers and incubated in the Growth profiler instrument (Enzyscreen) at 16°C with 190 rpm shaking speed. The cameras were set to capture an image of the plates every 30 minutes. To observe biomass accumulation the images were analyzed by the software provided by the manufacturer (Enzyscreen).

### Fermentation conditions

For larger scale 3 ml of overnight YPD yeast culture was inoculated into 30 ml of wort in 100 ml shake flasks with foam plug and grown for 2 additional days to generate a starter yeast culture. The starter culture was transferred into 250 ml glass bottles with wort in total volume of 160 ml. The final cell concentration was 1-1.5x10⁷ cells per ml. The fermentation was carried out anaerobically with stirring (130 rpm) at 16°C unless otherwise indicated. Gas production was monitored using the Ankom RF gas production system (Ankom Technology). After six days, samples were collected by centrifugation and the flavor profiles were analyzed.

### Sporulation

Overnight yeast culture was patched onto sporulation plates (as described under *Yeast growth conditions* above and in Lundblad and Struhl, 2008). The cultures were inspected under a microscope for the presence of asci, typically formed within 5 days at room temperature. Asci were treated with 50 µl of Zymolyase (US Biological) solution (0.5 mg/ml) for 20 min at 30°C during moderate shaking. 400 µl of sterile water was added and the treated asci were dropped onto an YPD plate. Spores were isolated using a dissection microscope (MSM400, Singer Instruments).

### Mating type analysis of spores - Halo assay

After spore isolation the mating type was observed via halo assay on YPD plates with 0.05% Triton X with a lawn (dense culture with no single colonies) of a mating pheromone sensitive (PS) strain (either α-PS or a-PS, Table 1). The growth inhibitory zone of the PS strains around an analyzed strain was visually scored after 2 days of growth. The growth inhibition of the analyzed strains on the pheromone sensitive (PS) strain is a response to the pheromone emitted by the analyzed strain of opposite mating type (see also paragraph 2.5 of Kempf et al., 2017 Microbiological Research 200:53-63).

### Genetic crossing and hybrid selection

Genetic crossing was performed by standard techniques as described in (Treco and Winston, 2008). Briefly, two different overnight cultures (Table 1) of equal concentrations (5x10⁶ cells/ml) were mixed in 1:1 ratio and patched on YPD plates. After approximately 12 hrs of mating, cells were scraped from the plates and diluted to the concentration of ca 4x10³ cells per ml and re-plated on agar plates.

For detection of hybrids colonies, clones were plated on X-gal selection plates as described in the Materials and Methods above. This results in blue colonies if *S. eubayanus* is present in the clone.

Hybrids of *S. eubayanus* and *S cerevisiae* were selected at 37 °C, since pure *S. eubayanus* cannot grow at this temperature. Blue colonies growing at 37 °C are therefore hybrids.

Hybrids of *S. eubayanus* and lager yeast spore *(S. pastorianus)* were selected on SG plates with sulfometuron methyl grown at room temperature. Hybrid colonies can grow on SG with sulfometuron methyl (lager spore feature) and are blue (S. *eubayanus* feature).

### Mutagenesis conditions

The yeast strains were grown in 12-ml culture tubes overnight. The culture was collected by centrifugation, washed twice with PBS + 1mM EDTA buffer and 10⁸ cells in 1 ml buffer were used for mutagenesis. The number of cells were determined by Cellometer (Nexcellom Biosciences) according to the manufacturer's instructions. The mutagenesis was performed using 3% ethyl methanesulfonate (EMS) as described in Winston, 2008. The mutagenesis time was determined for each strain by calculation the number of colonies surviving on YPD plates treated with EMS divided by the number of colonies on control (non-treated) plates. The survival rate was chosen to be between 45-65%. The efficiency of the mutagenesis procedure was evaluated by plating the treated and untreated cultures on plates with canavanine (60 mg/l).

### Analysis of flavor profiles in fermented wort samples

Gas chromatography was used to determine the concentration of volatile compounds in the samples, such alcohols (propanol, isobutanol, isoamyl alcohol, 2-phenylethanol), acids (caproic acid, caprylic acid, capric acid), esters (ethylacetate, isobutylacetate, isoamyl acetate, ethyl caproate, ethyl caprylate, ethyl butyrate and 2-phenylethyl acetate) and phenols (4-vinylguaiacol). The volatiles were extracted from wort samples with carbon disulfite (CS2). The subsequent qualitative analysis was performed using Gas chromatography with a Flame Ionization Detector (GC-FID). Agilent 6890A gas chromatograph with a split/splitless injector and a 30m x 0,32mm x 0,25µm DBWAX capillary column was used for the analysis. The sample extract was introduced to the preheated injector at 250°C, where it was volatilized and lead to the column with helium as carrier gas. In the flame ionized detector, the components were led through an air-hydrogen flame at 250°C that decomposed the component due to the high temperature. For determination of diketones Head Space Gas Chromatography with Electron Capture Detection (HS GC-ECD) was used. A gas chromatograph with a head space injector, a CP-Wax 60m x 0,25mm x 0,39mm capillary column and an electron capture detector with nickel-63 as β-emitter was used for the analysis. The head space samples were collected from the sample vials by the head space injector at 105°C as small amounts of head space gas and introduced to the column. The head space here is defined as the gas between the sample surface (head) and the top of the closed vial. An argon/methane mixture was used as carrier gas. Data analysis was performed using Chemstation software. Due to the instability of 4-VG standard, 4-VG values were calculated as 4-VG peak area divided by peak area of internal standard (octanol). The final values were displayed as relative values (related to POF negative lager yeast strain). Alcohol and real degree of fermentation (RDF) were measured using the Alcolyzer Beer Analyzing System consisting of DMA 4500 M density meter and Alcolyzer Beer ME measuring module (Anton Paar) according to the manufacturer's instructions.

### Example 1 - Generation of heterothallic S. eubayanus strain

In order to generate a heterothallic *S. eubayanus* strain, a *S. eubayanus strain* with wild type HO alleles (CBS 12357) was subjected to random mutagenesis, creating a library of mutagenized *S. eubayanus* cells, which was screened using droplet digital PCR for the presence of a non-sense (C238T) mutation in SeHO gene.

### Library preparation

*S. eubayanus* CBS12357 cells were treated with EMS as described in the material and method section under Mutagenesis conditions. A mutagenesis time of 60 minutes giving survival rate of 55-60% was used. This procedure led to the generation of a library of mutagenized *S*. *eubayanus cells.* The cells from the mutagenized library were diluted in YPD medium and aliquoted into a 96-well plate in a concentration of approximately 2500 viable cells in 100 µl aliquots (taking into account survival ratio of the population after the treatment). The library was grown for two days, copied by taking 10 µl of the saturated culture into 90 µl YPD and original library stored after adding 100 µl of 50% glycerol into each well. The copied culture was grown overnight and used for DNA isolation.

### Digital droplet PCR analysis (ddPCR)

DNA was isolated using MasterPure^{™} Yeast DNA Purification Kit (Lucigen). Digital PCR technology was used for identification of the particular mutation in the mutagenized population. PrimePCR assays consisting of TaqMan PCR probes and primers were designed using the Bio-Rad design tool (Bio-Rad) to distinguish between two different nucleotides at the same location in a gene of interest (for the HO mutation the target sequence provided in Table 2 was used). A master mix of the TaqMan assay solution, DNA and PCR reagents (Bio-Rad SuperMix) was separated into droplets using the QX200 AutoDG Droplet Digital PCR system (Bio-Rad), according to the manufacturer's instructions. After the PCR reaction, the droplets were analyzed using the QX200 instrument (Bio-Rad). The difference in abundance between two different nucleotides at the same position in the gene was calculated based on the amplitude of the signals of the TaqMan assays using the software analysis tool Quantasoft (Bio-Rad). The mutagenized population of yeast cells in a well where the mutation was identified was grown for 6 hrs in an Eppendorf tube with 0.5 ml YPD medium with shaking. The cell culture was diluted and aliquoted in the final concentration of 50-100 cells per well and analyzed using ddPCR as described above. Finally, the culture from the well with the present mutation was plated on YPD plates and 96 colonies randomly picked into a microtiter plate using the QPix 400 colony picker (Molecular Devices). The method for identifying yeast cells with a specific mutation employing ddPCR is also described in WO 2018/001884, which is hereby incorporated be reference.

**Table 2. PCR probes and primers used in the TaqMan analysis**

| **Customiz ed assay Bio-Rad ID** | **Target gene** | **Mutation** | **Translation** | **Target sequence (Bio-Rad design tool input sequence)** | **Genebank gene ID accession no.** |
|---|---|---|---|---|---|
| dMDS542254955 | *S. eubayanus* HO | C238T | Q80STOP | >HO_Q80_STOP_CA A-TAA (SEQ. ID. NO. 8) | 28929993 |
| dMDS354068073 | *S. eubayanus FDC1*-like | G477A | W159STOP | >EuFDC1_W159_ST OP_TGG-TGA (SEQ. ID. NO. 9) | 28933553 |
| dMDS742707788 | *S. cerevisiae FDC1* | G513A | W171STOP | >FS_FDC1_171Stop2 (SEQ. ID. NO. 1 0) | 852152 |

The ddPCR was designed to identify a mutation in the HO gene of S. *eubayanus* containing a substitution mutation from cytosine (C) to thymine (T) at nucleobase position 238 in the HO coding sequence (SEQ. ID. NO: 1)(Fig. 1A). This mutation introduced a premature stop codon into the coding sequence resulting in an amino acid sequence where the glutamine (Q) in position 80 is converted to a stop codon, leading to a truncated HO endonuclease which is expected to be inactive.

A strain with the C238T mutation was isolated from the single colonies. The isolated strain was referred to as S.e. ho/HO (see Table 1). The fractional abundance of the mutation of the clonal population was at 50% corresponding to the diploid nature of the strain suggesting that one allele of the gene was affected in the isolated strain.

After spore isolation from S.e. ho/HO the phenotype was observed via the halo assay described in the materials and method section. The spores causing growth inhibition of the PS strains were selected and genotype confirmed by ddPCR proving that the presence of the mutation with 100% abundance (Fig. 1B).

A schematic representation of the experimental procedure can be viewed in Figure 2.

Acquired S.e. ho^{C238T} spores were tested for growth in wort in 96-well plates in anaerobic conditions at 16°C. There was no significant difference among spores from different asci pointing at quite homozygous nature of the yeast (see Fig. 3).

These results demonstrate a successful isolation of *S. eubayanus* mutants with the specified non-sense mutant in the HO gene of S. *eubayanus,* which causes inability of the yeast cell to switch mating type, thus enabling the possibility to keep pure haploid lines.

### Example 2. Heterothallic S. eubayanus facilitates breeding.

The S. *eubayanus* spores named S.e. ho a or α generated in example 1 from the S.e. ho/HO strain were crossed with spores of the opposite mating type selected from one of the following strains: haploid *S. cerevisiae* strain S.c. PE-2 a or S.c. PE-2 α (Table 1) or a *S. pastorianus* yeast lager spore a (Table 1) to produce novel lager strains via cell to cell mass mating. The genetic crossing and hybrid selection was performed as described in the materials and method section.

The strains were successfully mated. A strain was selected from the crossing of haploid HO negative S. *eubayanus* yeast cells with haploid PE-2 *S. cerevisiae* strain and named novel lager 1 (NL1). Likewise, a strain of the crossing of haploid HO negative S. *eubayanus* yeast cells with lager yeast spore *(S. pastorianus)* was selected and named novel lager 2 (NL2).

The hybridization frequency was between 19 - 26% across 3 experiments for NL 1 and between 10 and 16 % for NL2 (Fig. 4). The hybridization frequency for NL1 was calculated as the amount of blue colonies (hybrids) grown at 37°C per total amount of colonies grown at room temperature. In the cases of *S. eubayanus* spores mating with *S. cerevisiae* spores almost every second colony grown at 37°C (non-permissive temperature for *S. eubayanus*) was a hybrid clone (Fig. 4). The hybridization frequency for breeding between *S. eubayanus* spores and lager spore clone (*S. pastorianus*) was determined from the amount blue colonies surviving on SG plates with X-gal and sulfometuron methyl divided by the total number of colonies on SG plates.

The results show that the frequency of hybridization with *S. cerevisiae* partners is sufficient to avoid requirements for selection or laborious high throughput screenings. Instead, natural features of the strains such as growth at 37°C or galactosidase activity can be used to visualize the hybrids without the use of fluorescent staining and advanced equipment. If natural selective markers are not available, standard PCR techniques can be used for hybrid confirmation without any excessive workload. In conclusion, the development of haploid S. *eubayanus* yeast cells greatly facilitates breeding and creating of diverse hybrids, as well as providing a simple platform for potential tailor-made changes in the genome.

### Example 3. Heterothallic S. eubayanus brings flavor diversity into lager yeast portfolio

Selected NL1 and NL2 hybrids as well as parental strains S. *eubayanus CBS12357, S. cerevisiae PE-2,* and *S. pastorianus lager* yeast (Table 1) were examined for fermentation properties and flavor profiles in 160 ml wort at 16°C as described in the materials and method section under fermentation conditions. CO₂ production was monitored using a gas pressure monitoring system during fermentation at either 10 °C or 16 °C.

The flavor profile of the fermentations was analyzed as described in the materials and method section. The results are shown in Table 3.

**Table 3. Flavor profile of the parent strains S. eubayanus, S. cerevisiae, S. pastorianus and the hybrids Novel lager 1 and Novel lager 2. The numbers are the average of 3 or 4 replicates. 4-VG value is displayed as a relative value related to the value of POF negative S.p. lager yeast**

| **Average levels** | **S. e. *CBS12357*** | **S.c. PE-2** | **S.p. Lager yeast** | **Novel lager 1** | **Novel lager 2** |
|---|---|---|---|---|---|
| **Alcohol (% V/V)** | 4,85 | 6,25 | 7,46 | 5,76 | 6,96 |
| **RDF (%)** | 46,74 | 64,09 | 72,12 | 62,57 | 70,17 |
| **Acetaldehyde, mg/l** | 13,67 | 3,06 | 1,14 | 1,36 | 1,40 |
| **Ethylacetate, mg/l** | 11,67 | 12,20 | 20,05 | 14,82 | 22,37 |
| **Propanol, mg/l** | 20,32 | 31,33 | 40,25 | 23,13 | 30,99 |
| **Isobutanol, mg/l** | 27,78 | 28,48 | 18,85 | 25,42 | 20,94 |
| **Isoamylacetate, mg/l** | 1,27 | 1,46 | 2,90 | 1,66 | 2,82 |
| **Isoamyl alcohol, mg/l** | 115,10 | 91,47 | 77,36 | 127,07 | 97,72 |
| **Ethyl caproate, mg/l** | 0,06 | 0,20 | 0,07 | 0,11 | 0,05 |
| **Isobutylacetate, mg/l** | 0,10 | 0,18 | 0,12 | 0,15 | 0,14 |
| **Ethyl butyrate, mg/l** | 0,03 | 0,05 | 0,04 | 0,05 | 0,05 |
| **Caproic acid, mg/l** | 0,42 | 3,32 | 1,80 | 2,40 | 1,30 |
| **2-Phenylethanol, mg/l** | 62,69 | 60,12 | 58,47 | 72,21 | 61,13 |
| **Caprylic acid, mg/l** | 0,30 | 4,88 | 1,86 | 3,05 | 1,30 |
| **Ethyl caprylate, mg/l** | 0,03 | 0,39 | 0,20 | 0,32 | 0,12 |
| **2-Phenylethyl acetate, mg/l** | 0,98 | 0,70 | 1,22 | 1,00 | 1,41 |
| **Capric acid, mg/l** | 0,61 | 1,71 | 0,35 | 1,65 | 0,31 |
| **Sum Acids, mg/l** | 1,33 | 9,91 | 4,01 | 7,09 | 2,91 |
| **Sum Heavy Volatile Alcohols, mg/l** | 110,79 | 119,93 | 117,57 | 120,75 | 113,05 |
| **Sum Esters, mg/l** | 14,14 | 15,17 | 24,61 | 18,11 | 26,96 |
| **4-VG (relative value)** | 13,03 | 12,98 | 1 | 12,175 | 9,354 |
| **Total Diacetyl, µg/l** | 154,00 | 43,67 | 17,67 | 80,75 | 40,25 |

From the gas accumulation curve it is apparent that wild type *S. eubayanus* did not ferment all the sugars in wort and did not finish fermentation before the experiment was ended, Fig. 5B. Furthermore, *S. eubayanus* reached low levels of RDF (below 50%) as well low alcohol levels (below 5%), but produced substantially high level of fusel alcohols (isoamylalcohol, isobutanol, 2-phenylethanol). The level of common esters (isoamyl acetate, phenylethyl acetate, ethyl acetate, ethyl hexanoate) was low and there was a high concentration of off-flavors such as 4-vinylguaiacol (4-VG) and diacetyl.

Poor fermentation properties and ester formation could be compensated by crossing with a *S. cerevisiae* parent. The production of CO₂ can be correlated with the uptake of sugar by the yeast strains. Figure 5 shows the CO₂ accumulation curves for the yeast strains of the disclosure fermenting wort at 10 °C and 16°C. As maltose is the most abundant sugar in wort, this suggests that the hybrid strain, novel lager 1 has improved maltose uptake. Likewise, alcohol production and final RDF value increased compared to the parent *S. eubayanus.* The hybrid strain novel lager 2 also showed higher production of all common esters, the level of isoamyl acetate, ethyl acetate and phenylethyl acetate was even higher when compared to the parent *S. cerevisiae* strain, while keeping high levels of aromatic alcohols (isoamyl alcohol and 2-phenylethanol) and fatty acid-derived esters (ethyl hexanoate, a feature typical for the particular *S. cerevisiae* strain).

The novel lager 2 hybrid had increased RDF compared to the parent *S. eubayanus and S. cerevisiae* strains and the NL1 hybrid (though slightly lower than the control *S. pastorianus* lager strain). The esters levels of ethyl acetate, phenylethyl acetate and ethyl butyrate, were higher as well as production of some alcohols (but not too high) even when compared to the *S. pastorianus* lager yeast strain.

The fermentation was repeated at 10°C, evaluating sugar fermentation in terms of the gas accumulation curve (Fig. 5A) and the RDF and alcohol %, shown in Table 4.

**Table 4. Alcohol and RDF of parent strains S. eubayanus, S. cerevisiae, S. pastorianus and the hybrids Novel lager 1 and Novel lager 2 after fermentation at 10°C. The numbers are the average of 2 replicates.**

| **Strain** | **Alcohol (v/v %)** | **RDF %** |
|---|---|---|
| S.p. lager yeast | 6,58 | 69,12 |
| S.c. PE-2 | 6,44 | 61,00 |
| S.e. CBS12357 | 5,91 | 54,86 |
| NL1 | 6,60 | 62,35 |
| NL2 | 7,43 | 69,03 |

Interestingly, the novel lager 1 and novel lager 2 hybrid strains both outperformed the *S. pastorianus* lager strain and parent *S. cerevisiae* strain in terms of fermentation performance at 10°C as they finished fermentation before the control lager strain. This is demonstrated in Figure 5A, which displays the CO₂ accumulation of the yeast strains. Furthermore, novel lager hybrid 2 reaching comparable RDF to the control lager strain but higher alcohol level.

The present invention provides novel yeast hybrids, which broaden the range of fermentation temperature outperforming the parental strains at 10°C. In addition, the flavor profiles change as parent *S. eubayanus* serves as a rich source of fusel alcohols, while parent *S. cerevisiae* counterpart provides the capacity to produce esters and wort fermentation capabilities, in terms of more efficient sugar consumption. In general, the novel hybrids produce higher concentrations of, in particular, acetate esters, compared to their respective parents.

### Example 4. Further mutagenesis to eliminate phenolic off-flavours in novel hybrids

Wild non-brewer's *Saccharomyces* strains are responsible for production of 4-VG (considered as undesired clove-like flavor in majority of beer styles) by enzymatic conversion of ferulic acid in wort. Inactivation of *FDC1* is known to abolish the production of 4-VG, resulting in a yeast strain without phenolic off-flavors (POF negative).

For the purpose of generating novel inter species POF negative hybrids of S. *eubayanus* and S. *cerevisiae* the bioethanol strain S.c. PE-2 and the S.e. ho a and S.e. ho α spores from Example 1 were subjected to mutagenesis and screening by digital PCR as described in Example 1. With an adaptation of the mutagenesis time to 20 minutes for the S.c. PE-2 cells, whereas the mutagenesis time of 60 min was maintained for the S.e. cells.

For the digital PCR the assays named dMDS742707788 (S.c.) or dMDS354068073 (S.e.) were used to identify non-sense mutations in *FDC1* gene leading to an introduction of a premature STOP codon in the *FDC1* nucleotide sequence. Specifically, for the *S*. *cerevisiae fdc1* gene a stop codon at nucleotide position 513 of SEQ. ID. NO: 6 changing guanine(G) to adenine(A) was identified, and for the *S. eubayanus fdc1* gene a stop codon at nucleotide position 477 of SEQ. ID. NO: 4 changing guanine(G) to adenine(A) was identified. Genotyping of the single colonies of PE-2 α (*fdc1*^{G513A}) by ddPCR proved the presence of the mutation with 100% abundance (Fig. 6).

The phenotype was confirmed by fermentation in 250 µl of wort in 96-well deep-well plates in YPD supplemented with 1 mM ferulic acid as described in the materials and method section under yeast growth conditions. The supernatant absorbance was measured at 325 nm using a UV spectrophotometer (Tecan) (Mertens *et al.,* 2017). The relative absorbance at 325 nm of the *S. eubayanus* samples in YPD medium with 1mM ferulic acid are shown in Table 5.

**Table 5: Average absorbance at 325 nm (N=3) of fermented samples divided by the value of non-inoculated sample of YPD with 1mM ferulic acid.**

| **Strain** | **Absorbance 325 nM** |
|---|---|
| S.e. CBS12357 | 0,57 |
| S.e. ho a | 0,50 |
| S.e. ho α | 0,48 |
| S.e. fdc1 a | 0,95 |
| S.e. fdc1 α | 0,98 |

Likewise, a MATa POF negative spore of the *S. cerevisiae* strain was prepared by breeding of the mutagenized MATα spore with a wild type spore of the opposite mating type and subsequent sporulation, tetrad dissection and selection of a spores with the POF negative phenotype using the assay described above.

The selected spores were named S.c. PE-2 a fdc1 and S.c. PE-2 α fdc1 for the S. cerevisiae mutants and S.e. fdc1 a and S.e. fdc1 α for the *S. eubayanus* mutants.

The POF negative spores were crossed as describe in Example 2 using the following combinations:
S.c. PE-2 a fdc1 x S.e. fdc1 ho α obtaining the novel lager hybrid NL3
*S. pastorianus* lager yeast spore x S.e. fdc1 ho α obtaining the novel lager hybrid NL4 For detailed information about the strains, see Table 1.

The results are shown in Table 6 below.

**Table 6. Flavor profile of the POF negative hybrids, Novel lager 3 and Novel lager 4 and the POF positive Novel lager 1 and Novel lager 2 as well as the parental strains and the control strain S.p. Lager yeast. The numbers are the average of 2 or 3 replicates. 4-VG value is displayed as a relative value related to the value of POF negative S.p. lager yeast**

| | **S.p. lager yeast** | **S.e. CBS12357** | **S.c. PE-2** | **NL1** | **NL2** | **NL3** | **NL4** |
|---|---|---|---|---|---|---|---|
| **alcohol (%v/v)** | 7.77 | 4.85 | 6.52 | 6.40 | 7.37 | 6.61 | 7.44 |
| **RDF (%)** | 71.48 | 45.71 | 62.97 | 62.57 | 70.24 | 62.49 | 70.70 |
| **Propanol (mg/l)** | 35.86 | 16.89 | 33.73 | 24.59 | 31.60 | 24.47 | 36.30 |
| **Isobutanol (mg/l)** | 17.38 | 29.98 | 37.81 | 27.91 | 22.76 | 26.43 | 26.64 |
| **Isoamyl alcohol** | 74.02 | 142.95 | 83.73 | 124.28 | 105.02 | 127.65 | 99.55 |
| **2-Phenylethanol (mg/l)** | 36.76 | 57.85 | 52.28 | 54.52 | 46.58 | 49.39 | 44.42 |
| **Isoamylacetate (mg/l)** | 2.89 | 0.84 | 1.24 | 1.40 | 3.08 | 2.22 | 3.67 |
| **Ethylacetate (mg/l)** | 20.84 | 10.15 | 12.12 | 14.27 | 23.85 | 16.17 | 26.33 |
| **2-Phenylethyl acetate (mg/l)** | 0.84 | 0.68 | 0.44 | 0.82 | 0.95 | 0.81 | 0.98 |
| **Isobutyl acetate (mg/l)** | 0.10 | 0.05 | 0.11 | 0.08 | 0.09 | 0.09 | 0.09 |
| **Ethyl caproate (mg/l)** | 0.06 | 0.05 | 0.22 | 0.15 | 0.06 | 0.23 | 0.09 |
| **Ethyl caprylate (mg/l)** | 0.11 | 0.03 | 0.23 | 0.15 | 0.08 | 0.17 | 0.10 |
| **Ethyl butyrate (mg/l)** | 0.02 | 0.02 | 0.03 | 0.03 | 0.03 | 0.03 | 0.03 |
| **4-VG (relative value)** | 1 | 13.78 | 14.89 | 14.24 | 6.48 | 1.14 | 1.12 |
| **Total Diacetyl (ug/l)** | 16.00 | 223.00 | 61.00 | 60.50 | 32.50 | 35.67 | 25.67 |
| **Acetaldehyde (mg/l)** | 1.35 | 7.98 | 2.41 | 1.28 | 1.65 | 1.75 | 1.30 |

The resulting hybrid strains, NL3 and NL4 did not produce significant levels of 4-VG after fermentation in wort while keeping the production of other flavors at comparable levels to the FDC1 positive hybrids (NL1vs NL3 and NL2 vs NL4) (Table 6).

Disruption of the FDC1 gene results in lack of 4-VG production during fermentation, hence avoiding the off-flavour that 4-VG contributes too. These strains are therefore termed POF negative strains.

Essentially, Example 4 illustrates, the ease with which new properties can be introduced into a strain (e.g. NL1 and NL2) when both the spores which the parent strain originated from are *ho* negative. In this case one can go back to the ho negative S. eubayanus spore and the ho negative S. cerevisiae spore mutagenize these and then re-cross the spores and a predicable result can be achieved.

### Sequence overview

SEQ. ID. NO. 1
   Polynucleotide sequence of coding region of *S. eubayanus* HO gene (wild type).
SEQ. ID. NO. 2
   Amino acid sequence of *S. eubayanus* HO polypeptide (wild type).
SEQ. ID. NO. 3
   Polynucleotide sequence of coding region of *S. eubayanus* HO gene (mutant).
SEQ. ID. NO. 4
   Polynucleotide sequence of coding region of *S. eubayanus* FDC1-like gene (wild type).
SEQ. ID. NO. 5
   Amino acid sequence of *S. eubayanus* FDC1-like polypeptide (wild type).
SEQ. ID. NO. 6
   Polynucleotide sequence of coding region of *S. cerevisiae* FDC1 gene (wild type).
SEQ. ID. NO. 7
   Amino acid sequence of *S. cerevisiae* FDC1 polypeptide (wild type).
SEQ. ID. NO. 8
   Polynucleotide sequence of target sequence for mutant *S. eubayanus* HO gene in TaqMan analysis.
SEQ. ID. NO. 9
   Polynucleotide sequence of target sequence for mutant *S. eubayanus* FDC1-like gene in TaqMan analysis.
SEQ. ID. NO. 10
   Polynucleotide sequence of target sequence for mutant *S. cerevisiae* FDC1 gene in TaqMan analysis.

### References

Alexander, W.G., Peris, D., Pfannenstiel, B.T., Opulente, D.A., Kuang, M., Hittinger, C.T., 2016. Efficient engineering of marker-free synthetic allotetraploids of Saccharomyces. Fungal Genet Biol 89, 10-17. https://doi.org/10.1016/j.fgb.2015.11.002

Argueso J.L. et al. 2009 Genome structure of a Saccharomyces cerevisiae strain widely used in bioethanol production Genome Res. 19(12): 2258-2270. doi: 10.1101/gr.091777.109.

Baker E, Wang B, Bellora N, Peris D, Hulfachor AB et al. 2015. The genome sequence of Saccharomyces eubayanus and the domestication of lager-brewing yeasts. Mol Biol Evol 2015;32:2818-2831

Basso L.C., de Amorim HV, de Oliveira AJ, Lopes ML. 2008 Yeast selection for fuel ethanol production in Brazil. FEMS Yeast Res; 8:1155-1163. https://doi.ora/10.1111/j.1567-1364.2008.00428.x

Briggs, D. E., Stevens R, Young T.W., Hough J.S., 1981. Malting and Brewing science.

Gorter de Vries, A.R., Koster, C.C., Weening, S.M., Luttik, M.A.H., Kuijpers, N.G.A., Geertman, J.-M.A., Pronk, J.T., Daran, J.-M.G., 2019. Phenotype-Independent Isolation of Interspecies Saccharomyces Hybrids by Dual-Dye Fluorescent Staining and Fluorescence-Activated Cell Sorting. Front. Microbiol. 10. https://doi.org/10.3389/fmicb.2019.00871

Gunge, N and Nakatomi Y., 1972. Genetic Mechanisms of Rare Matings of the Yeast SACCHAROMYCES CEREVISIAE Heterozygous for Mating Type. Genetics 70(1): 41-58.

Hough, J. S., Briggs D.E., Stevens R., Young T.W., 1982. Malting and Brewing science: Hopped Wort and Beer, Volume 2.

Kempf C, Lengeler K, Wendland J, 2017. Differential stress response of Saccharomyces hybrids revealed by monitoring Hsp104 aggregation and disaggregation. Microbiological Research 200:53-63.

Krogerus, K., Magalhães, F., Vidgren, V., Gibson, B., 2015. New lager yeast strains generated by interspecific hybridization. J Ind Microbiol Biotechnol 42, 769-778. https://doi.org/10.1007/s10295-015-1597-6

Krogerus, K., Arvas, M., De Chiara, M., Magalhães, F., Mattinen, L., Oja, M., Vidgren, V., Yue, J.-X., Liti, G., Gibson, B., 2016. Ploidy influences the functional attributes of de novo lager yeast hybrids. Appl Microbiol Biotechnol 100, 7203-7222. https://doi.org/10.1007/s00253-016-7588-3

Libkind D, Hittinger C.T, Valério E, Gonçalves C, Dover J, Johnston M, Gonçalves P, Sampaio J.P, 2011. Microbe domestication and the identification of the wild genetic stock of lager-brewing yeast. PNAS 108(35):14539-14544. https://doi.org/10.1073/pnas.1105430108.

Lundblad, V and Struhl, K, 2008. Yeast. Current Protocols in Molecular Biology 82(1): 1934-3639. https://doi.org/10.1002/0471142727.mb1300s82.

Mertens, S., Steensels, J., Saels, V., De Rouck, G., Aerts, G., Verstrepen, K.J., 2015. A Large Set of Newly Created Interspecific Saccharomyces Hybrids Increases Aromatic Diversity in Lager Beers. Appl Environ Microbiol 81, 8202-8214. https://doi.org/10.1128/AEM.02464-1

Mertens, S., Steensels, J., Gallone, B., Souffriau, B., Malcorps, P., Verstrepen, K.J., 2017. Rapid Screening Method for Phenolic Off-Flavor (POF) Production in Yeast. Journal of the American Society of Brewing Chemists 75, 318-323. https://d0i.org/10.1094/ASBCJ-2017-4142-01

Naumov GI, 1996. Genetic identification of biological species in theSaccharomyces sensu stricto complex. Journal of Industrial Microbiology 17:295-302.

Needleman and Wunsch, 1970. A general method applicable to the search for similarities in the amino acid sequence of two proteins. J Mol Biol. 48(3): 443-453. doi: 10.1016/0022-2836(70)90057-4

Rice P, Longden I., Bleasby A, 2000._EMBOSS: the European Molecular Biology Open Software Suite. Trends Genet 16(6): 276-277. doi: 10.1016/s0168-9525(00)02024-2

Sanchez, R.G., Solodovnikova, N., Wendland, J., 2012. Breeding of lager yeast with Saccharomyces cerevisiae improves stress resistance and fermentation performance. Yeast 29, 343-355. https://doi.org/10.1002/vea.2914

Treco, D.A., Winston, F., 2008. Growth and manipulation of yeast. Curr Protoc Mol Biol Chapter 13, Unit 13.2. https://doi.org/10.1002/0471142727.mb1302s82

Winston, F., 2008. EMS and UV mutagenesis in yeast. Curr Protoc Mol Biol Chapter 13, Unit 13.3B. https://doi.org/10.1002/0471142727.mb1303bs82

## Claims

1. A method of generating hybrid yeast cells, the method comprising the steps of:
(a) Providing heterothallic *S. eubayanus* yeast cells, wherein said yeast cells comprise a mutation in the gene encoding an HO endonuclease, wherein the wild type HO endonuclease gene comprises the sequence of SEQ. ID. NO. 1 or a sequence sharing at least 80%, such as at least 90%, for example at least 95% sequence identity therewith and which encodes HO endonuclease of SEQ. ID. NO: 2 or a functional homologue thereof; and
(b) mating the *S. eubayanus* yeast of step (a) with yeast of the genus *Saccharomyces,* wherein said *Saccharomyces* has the opposite mating type of the *S. eubayanus* yeast;
thereby obtaining a hybrid yeast cell.

2. The method according to claim 1, wherein step (b) comprises mating spores of said S. *eubayanus* yeast with spores of said *Saccharomyces* yeast, wherein said spores of *Saccharomyces* has the opposite mating type of the spores of the *S. eubayanus* yeast.

3. The method according to any one of the preceding claims, wherein the yeast of the genus *Saccharomyces* is selected from the group consisting of *S. cerevisiae, S. pastorianus, S. kudriavzevii, S. bayanus, S. uvarum, Saccharomyces cariocanus* and *Saccharomyces mikatae.*

4. The method according to any one of the preceding claims, wherein the method further comprises a step of selecting hybrid yeast cells which are capable of producing increased levels of esters, for example increased levels of one or more esters selected from the group consisting of ethylacetate, ethylbutyrate and 2-phenylethyl acetate, compared with one or both of the parent *S. eubayanus* and *Saccharomyces* strains provided in step (a) and (b), respectively.

5. The method according to any one of the preceding claims, wherein the method further comprises a step of selecting hybrid yeast cells which have an increased real degree of fermentation (RDF) compared to the *S. eubayanus* yeast strain provided in step (a).

6. The method according to any one of the preceding claims, wherein the hybridization frequency of the S. *eubayanus* yeast strain of step (a) with the yeast of the genus *Saccharomyces* is at least 8%, such as at least 10%.

7. A *S. eubayanus* yeast cell, wherein said yeast cell comprises a mutation in the gene encoding an HO endonuclease, wherein the wild type HO endonuclease gene comprises the sequence of SEQ. ID. NO. 1 or a sequence sharing at least 80%, such as at least 90%, for example at least 95% sequence identity therewith, wherein the mutation results in loss of function of the protein encoded by mutated HO endonuclease gene.

8. The yeast cell according to claim 7, wherein the mutation introduces a premature stop codon in the coding region.

9. The yeast cell according to any one of claims 7 to 8, wherein the mutated HO endonuclease gene encodes a mutant HO endonuclease lacking at least the 100 most C-terminal amino acids, such as lacking at least the 200 most C-terminal amino acids, for example lacking at least the 300 most C-terminal amino acids, such as lacking at least the 400 most C-terminal amino acids, preferably lacking at least the 510 most C-terminal amino acids of SEQ. ID. NO: 2.

10. The yeast cell according to any one of claims 7 to 9, wherein the mutation is a mutation from C to T at the position corresponding to nucleotide 238 of SEQ. ID. NO. 1.

11. The yeast cell according to any one of claims 7 to 10, wherein said heterothallic *S. eubayanus* yeast cells further comprises a mutation in the FDC1-like gene, wherein the FDC1-like gene comprises the sequence of SEQ. ID. NO. 4 or a sequence sharing at least 80%, such as at least 90%, for example at least 95% sequence identity therewith.

12. A hybrid yeast cell comprising a mutation in the gene encoding an HO endonuclease, wherein the wild type HO endonuclease gene comprise the sequence of SEQ. ID. NO. 1 or a sequence sharing at least 80%, such as at least 90%, for example at least 95% sequence identity therewith, wherein the hybrid yeast cell is a hybrid of an *S. eubayanus* yeast cell and a *Saccharomyces* yeast cell, wherein the mutation results in loss of function of the protein encoded by mutated HO endonuclease gene.

13. The hybrid yeast cell according to claim 12, wherein the hybrid yeast is of the species S. *pastorianus.*

14. Use of the hybrid yeast cells obtained by the method according to any one of claims 1 to 6, and/or the hybrid yeast cell according to any one of claims 12 to 13, for the production of a beverage, for example a beverage selected from the group consisting of beer, cider and wine.

15. A method of preparing a beverage, the method comprising the steps of:
(a) Providing an aqueous extract of malt and/or cereal kernels;
(b) providing the hybrid yeast cells obtained by the method according to any one of claims 1 to 6, and/or the hybrid yeast cell according to any one of claims 12 to 13;
(c) fermenting said aqueous extract with said yeast;
thereby obtaining said malt and/or cereal-based beverage.
